# EUROPEAN PATENT SPECIFICATION

(11) **EP 2 791 327 B1**
(45) Date of publication and mention of the grant of the patent: **30.08.2017**
(21) Application number: 12813297.4
(22) Date of filing: 12.12.2012
(51) Int. Cl.: C12N 9/10, C12P 19/44, C11D 1/00

(54) **FOAM ADSORPTION**
SCHAUMAUFNAHME
ADSORPTION DE MOUSSE

(30) Priority: 12.12.2011 EP 11192918
(43) Date of publication of application: 22.10.2014
(73) Proprietor: Amlika Mercantile Private Limited (AMPL), 400017 Mumbai (IN)
(72) Inventor: BLANK, Lars, Dortmund 44227 (DE); KUEPPER, Benjamin, 44894 Bochum (DE); DEL AMOR VILLA, Eva Maria, 41460 Neuss (DE); WICHMANN, Rolf, 44225 Dortmund (DE); NOWACKI, Christian, 44625 Herne (DE)
(74) Representative: Schiweck, Weinzierl & Koch Patentanwälte Partnerschaft mbB
(86) International application number: PCT/EP2012/075183
(87) International publication number: WO 2013/087674

(56) References cited:
- M. HEYD ET AL: "Continuous rhamnolipid production with integrated product removal by foam fractionation and magnetic separation of immobilized Pseudomonas aeruginosa", BIOTECHNOLOGY PROGRESS, vol. 27, no. 3, 1 May 2011 (2011-05-01), pages 706-716, XP055036355, ISSN: 8756-7938, DOI: 10.1002/btpr.607 cited in the application
- H.E. Reiling ET AL: "Pilot plant production of rhamnolipid biosurfactant by Pseudomonas aeruginosa.", Applied and environmental microbiology UNITED STATES May 1986, 31 May 1986 (1986-05-31), pages 985-989, XP055036395, Retrieved from the Internet: URL:http://aem.asm.org/content/51/5/985.fu ll.pdf [retrieved on 2012-08-27] cited in the application
- Clara Mata ET AL: "Foam Control using a Fluidized Bed of Hydrophobic Particles", , 31 October 1997 (1997-10-31), pages 1-23, XP055036397, University of Minnesota AEM, 107 Akerman Hall, 110 Union Street Minneapolis, MN 55455 Retrieved from the Internet: URL:http://www.aem.umn.edu/people/faculty/ joseph/archive/docs/mataddjfoam.pdf [retrieved on 2012-08-27]
- B. BURGHOFF: "Foam fractionation applications", JOURNAL OF BIOTECHNOLOGY, vol. 161, no. 2, 1 October 2012 (2012-10-01), pages 126-137, XP055036400, ISSN: 0168-1656, DOI: 10.1016/j.jbiotec.2012.03.008

## Description

### FIELD OF THE INVENTION

The present invention relates to methods for foam adsorption as described herein. In particular, the invention provides methods for the isolation of an amphipathic, a hydrophobic and/or a hydrophilic compound from a medium that is either hydrophilic or hydrophobic, respectively as claimed in the claims. These methods include allowing the formation and/or accumulation of foam, which includes the compound at the medium-gas interface, as well as applying the foam directly onto an adsorbent. The adsorbent effects collapse of the foam due to the removal of the amphiphatic, hydrophobic and/or hydrophilic compound from the foam lamella by adsorption. The method generally also includes isolating the adsorbed compound by consecutive desorption with a suitable solvent.

### BACKGROUND OF THE INVENTION

Increasing interest in biological surfactants has led to intensified research directed at more cost-efficient production of biosurfactants, relative to traditional surface-active components based on petrochemical feedstock. Biosurfactants are amphiphilic surface-active substances of microbial origin, i.e., synthesized by living cells. They are generally non-toxic, biodegradable and thus environmental friendly. Interest in microbial surfactants has been steadily increasing in recent years due to their diversity, environmentally friendly nature, possibility of large-scale production, selectivity, performance under extreme conditions and potential applications in environmental protection.

Main classes of biosurfactants are glycolipids, lipopeptides, phospholipids, and fatty acids (Banat et al. (2000), Appl Microbial Biotechnol 53:495-508). Biosurfactants have two ends namely, a hydrocarbon part which is less soluble in water (hydrophobic end). The hydrophobic part of the molecule is a chain of fatty acids, hydroxyl fatty acids, hydroxyl fatty acids or α-alkyl-β-hydroxy fatty acids. The water soluble end (hydrophilic) can be a carbohydrate, amino acid, cyclic peptide, phosphate, carboxylic acid or alcohol. The unique properties of biosurfactants allow their use and possible replacement of chemically synthesized surfactants in a number of industrial operations. In particular, these properties render biosurfactants capable of reducing surface and interfacial tension and forming microemulsions where hydrocarbons can solubilize in water or where water can solubilize in hydrocarbons. Such characteristics confer excellent detergency, emulsifying, foaming, and dispersing traits, which makes biosurfactants some of the most versatile process chemicals.

Biosurfactants are thus commercially important compounds with potential for use in environmental protection, petroleum, food, pharmaceutical, cosmetic and other industries. Typical applications of biosurfactants include herbicides and pesticides formulations, detergents, health care and cosmetics, pulp and paper, coal, textiles, ceramic processing and food industries, uranium pre-processing or mechanical dewatering of peat. Also, given the fact that the use of chemicals for the treatment of a hydrocarbon polluted site may contaminate the environment with their by-products, biological treatment may, however, efficiently destroy pollutants, while being biodegradable themselves. Hence, biosurfactant producing microorganisms may play an important role in the accelerated bioremediation of hydrocarbon contaminated sites.

The biosurfactant production depends on the fermentation conditions, environmental factors and nutrient availability. Typically, submerged microbial fermentation is appropriate for the production of biosurfactants, since it provides a degree of economic efficiency. The process can be operated in batch, fed-batch or continuous mode. However, at present, the use of biosurfactants has been limited by their high production costs and thus for many applications they cannot compete with chemically synthesized surfactants. Another limiting factor in biosurfactant production is their inhibitory effect on their own synthesis.

Accordingly, academia and industry have undertaken efforts to optimize biosurfactant production with the aim of minimizing costs and energy and maximizing biosurfactant recovery. More specifically, it has been shown that that two-phase partitioning systems with *in situ* extraction of the surfactant-enriched phase can accelerate biosurfactant production (Drouin and Cooper (1992), Biotech Bioeng 40:86-90). Other recovery methods that can be applied repeatedly are foam fractionation (Davis et al. (2001), Enzyme Microb Technol 28:346-354; Noah et al. (2002), Appl Biochem Biotech 98-100:803-813; Chen et al. (2006), J Chem Technol Biotechnol 81:1915-1922; Glazyrina et al. (2008), Appl Biochem Biotech 81:23-31; Heyd et al. (2008), Anal Biochem Chem 391:1579-1590; Sarachat et al. (2010), Biores Technol 101:324-330; Heyd et al. (2011), Biotechnol Prog 27(3):706-716), ultrafiltration (Sen and Swaminathan (2005), Process Biochem 40:2953-2958), and adsorption-desorption processes on polystyrene resins or activated carbon (Reiling et al. (1986), Appl Environ Microbiol 51:985-989; Dubey et al. (2005), Biotech Bioeng 40:86-90) as well as crystallization (Desai and Banat (1997), Microbiol Mol Biol Rev 61(1):47-64).

However, the recovery methods thus far applied in the art have one or more drawbacks. For example, though Chen et al. (2006) were able to continuously produce surfactin, a surfactant from *B. subtilis,* with foam fractionation, their recovery was low, since the fermenter volume was limited, and they had to mechanically collapse the fractionated foam - two limiting factors. Sarachat et al. (2010) had to separate *P. aeruginosa* cells before recovering the rhamnolipid biosurfactant and also had to chemically collapse the fractionated foam - a time and energy consuming process which also affected Glazyrina et al. (2008). These authors applied the so-called flounder, a device that enables foam fractionation after the biosurfactant production phase. Indeed, the production volume was low and the cells had to be separated from the foam produced before recovering the same. Heyd et al. (2011) propose a continuous biosurfactant production with integrated removal by foam fractionation and magnetic separation of immobilized bacterial cells which would otherwise be lost during foam fractionation. Specifically, Heyd et al. (2011) wish to retain immobilized bacterial cells which were entrapped in magnetic alginate beads. More specifically, high-gradient magnetic separation allowed back-flushing bacterial cells into the reactor. The foam collected by Heyd et al. (2011) was collapsed by the addition of citric acid. Reiling et al. (1986) proposed a combination of several purification processes including adsorption and ion exchange chromatography. In particular, production medium is separated from bacterial cells and applied to adsorption and ion exchange chromatography columns. This process is very elaborate and impractical for industrial scale biosurfactant production. Clara Mata et al, Foam Control using a Fluidized Bed of Hydrophobic Particles, 31 October 1997, pages 1-23, University of Minnesota ARM, URL: http://www.aem.umn.edu/people/faculty/joseph/archive/docs/mataddjfoam.pdf, discloses the use of hydrophobic particles for foam control.

In sum, thus far, the keystone in the recovery and also purification of biosurfactants is the separation of the production medium from the microorganisms as well as the reduction of the liquid volume. In batch-wise biosurfactant separation, microorganisms are generally removed by centrifugation prior to further recovery and purification steps. A preliminary purification step can be performed by precipitation, solvent extraction, or selective crystallization. When a biosurfactant is continuously separated from production medium, adsorption and/or ion exchange chromatography for recovering a biosurfactant from culture medium after separation of cells, membrane filtration or foam fractionation can be applied.

However, none of these processes allows continuous and sustainable biosurfactant production, because of not meeting industrial-size, wasting time and energy and being generally costly, for example, because of highly sophisticated technical equipment such microorganisms embedded in magnetic particles which are retained during foam fractionation by high-gradient magnetic separation. Also, none of the thus-far known processes has an appropriate solution how to easily process the foam comprising the desired biosurfactant. Mostly, it is suggested to collapse the foam mechanically, either forced or statistically, or chemically. Yet, these measures are either time or space consuming, or require energy and chemicals.

That being said, since most biosurfactants are amphipathic, there is a general need in the isolation of an amphipathic by way of a convenient, sustainable isolation and/or purification process. The isolation may be from medium, aqueous solutions and the like. However, a convenient, sustainable isolation and/or purification process is not only desirable for amphipathic compounds, but also for hydrophobic compounds from a hydrophilic medium or for hydrophilic compounds from hydrophobic medium, respectively.

Pondering the above, there is still a need to provide a convenient, sustainable isolation and/or purification process of amphipathic compounds, in particular biosurfactants, hydrophobic compounds from hydrophilic medium and/or hydrophilic compounds from hydrophobic medium.

Accordingly, the technical problem of the present invention is to comply with this need. This problem is solved by the methods of the independent claims.

### SUMMARY OF THE INVENTION

The present invention provides methods that allow isolating an amphipathic compound from a hydrophobic or hydrophilic medium, a hydrophilic compound from a hydrophobic medium as well as a hydrophobic compound from a hydrophilic medium. In a process of the invention foam containing the compound to be isolated is contacted with a suitable adsorbent. Depending on the chemical nature of the compound to be isolated, being hydrophilic, hydrophobic or amphiphilic, a suitable adsorbent may be selected. A suitable adsorbent is an adsorbent that assists, induces or brings about the collapse of foam that contains the compound. As illustrative examples, where the compound is hydrophilic, a hydrophobic adsorbent may be chosen, and where the compound is hydrophobic, a hydrophilic adsorbent may be chosen. In embodiments of a method of the invention where a microorganism such as a cell is employed, the microorganism need not be removed before contacting a respective foam with the adsorbent. The compound included in the foam is allowed to be removably/reversibly immobilized on the adsorbent. Desorption may for instance be achieved using a suitable fluid, e.g. a liquid. If a hydrophobic adsorbent is used, it may in particular embodiments be desirable to achieve desorption by contacting the adsorbent, on which the compound is reversibly immobilized, with a hydrophilic fluid. Thereby, in particular if a liquid is used for desorption, the desired compound can typically be obtained in highly concentrated form. In embodiments where a hydrophilic adsorbent is used, it may in some embodiments be desirable to achieve desorption by contacting the adsorbent, on which the compound is reversibly immobilized, with a hydrophobic fluid.

According to a first aspect, the present invention provides a process for the isolation of an amphipathic compound from a hydrophobic or hydrophilic medium. The process includes allowing the formation and/or accumulation of foam that includes the amphipathic compound at the hydrophobic or hydrophilic medium-gas interface, respectively. Further the process includes applying the foam directly onto an adsorbent, which is in the form of particles and effects collapse of the foam. The process also includes isolating the adsorbed amphipathic compound by desorption. In said process, the adsorbent is hydrophobic if the solvent of the culture broth is hydrophilic. Alternatively, in said process, the adsorbent is hydrophilic if the solvent of the culture broth is hydrophobic.

According to some particular embodiments of the process according to the first aspect, the process further includes one or more further steps of purifying the amphipathic compound.

In some embodiments of the process according to the first aspect the amphipathic compound is a biosurfactant.

In some embodiments of the process according to the first aspect the amphipathic compound is produced by culturing a host cell capable of producing the biosurfactant. In some embodiments the host cell is cultured. A host cell is in some embodiments grown at a temperature above 30°C.

In some embodiments the host cell is a prokaryotic cell. In some embodiments the prokaryotic cell is a gram-negative bacterial cell. In some embodiments the host cell is non-pathogenic for humans. In some embodiments the cell includes a *rhlA* gene or an ortholog thereof as well as a *rhlB* gene or an ortholog thereof. The *rhlA* gene or the ortholog thereof and/or the *rhlB* gene or ortholog thereof may be under the control of a heterologous promoter. The cell may further include a *rhlC* gene or an ortholog thereof. The *rhlC* gene or the ortholog thereof may in some embodiments be under the control of a heterologous promoter. The bacterial cell may in some embodiments be selected from the group consisting of *Pseudomonas putida, Pseudomonas chlororaphis, Pseudomonas fluorescens, Pseudomonas alcaligenes, Pseudomonas aeruginosa, Pseudomonas cepacia, Pseudomonas clemancea, Pseudomonas collierea, Pseudomonas luteola, Pseudomonas stutzeri, Pseudomonas teessidea, Escherichia coli, Renibacterium salmoninarum, Cellulomonas cellulans, Tefragenococcus koreensis, Burkholderia glumae, Burkholderia mallei, Burkholderia pseudomallei, Burkholderia plantarii, Burkholderia thailandensis, Acinetobacter calcoaceticus, Enferobacter asburiae, Enterobacter hormaechei, Pantoea stewartii* and *Pantoea ananatis.*

In some embodiments of the process according to the first aspect the amphipathic compound is a biosurfactant, which is produced by culturing a host cell capable of producing the biosurfactant. The host cell is in some embodiments a prokaryotic cell. In some embodiments the prokaryotic cell is a gram-negative bacterial cell. In some embodiments the host cell is non-pathogenic for humans. In some embodiments the cell includes a *rhlA* gene or an ortholog thereof as well as a *rhlB* gene or an ortholog thereof. The *rhlA* gene or the ortholog thereof and/or the *rhlB* gene or ortholog thereof may be under the control of a heterologous promoter. The cell may further include a *rhlC* gene or an ortholog thereof. The *rhlC* gene or the ortholog thereof may in some embodiments be under the control of a heterologous promoter. The bacterial cell may in some embodiments be selected from the group consisting of *Pseudomonas putida, Pseudomonas chlororaphis, Pseudomonas fluorescens, Pseudomonas alcaligenes, Pseudomonas aeruginosa, Pseudomonas cepacia, Pseudomonas clemancea, Pseudomonas collierea, Pseudomonas luteola, Pseudomonas stutzeri, Pseudomonas teessidea, Escherichia coli, Renibacterium salmoninarum, Cellulomonas cellulans, Tetragenococcus koreensis, Burkholderia glumae, Burkholderia mallei, Burkholderia pseudomallei, Burkholderia plantarii, Burkholderia thailandensis, Acinetobacter calcoaceticus, Enterobacter asburiae, Enterobacter hormaechei, Pantoea stewartii* and *Pantoea ananatis.* In some embodiments the host cell is cultured. The host cell may in some embodiments be grown at a temperature above 30°C.

In some embodiments of the process according to the first aspect is a process for the *in situ* isolation of a biosurfactant. The process includes culturing a host cell capable of producing the biosurfactant. The process further includes culturing allowing the formation and/or accumulation of foam that includes the biosurfactant at the culture broth-gas interface. Furthermore the process includes applying the foam directly onto an adsorbent which effects collapse of the foam. The process also includes isolating the adsorbed biosurfactant by desorption.

In some embodiments of the process according to the first aspect consists of the steps defined above. In such an embodiment the process consists of (a) allowing the formation and/or accumulation of foam that includes the amphipathic compound at the hydrophobic or hydrophilic medium-gas interface, respectively; (b) applying the foam directly onto an adsorbent, which effects collapse of the foam; and (c) isolating the adsorbed amphipathic compound by desorption.

In some embodiments the process further includes one or more further steps of purifying the amphipathic compound.

In some embodiments the process consists of (a) allowing the formation and/or accumulation of foam that includes the amphipathic compound at the hydrophobic or hydrophilic medium-gas interface, respectively; (b) applying the foam directly onto an adsorbent, which effects collapse of the foam; (c) isolating the adsorbed amphipathic compound by desorption; and (d) one or more steps of further purifying the amphipathic compound.

In some embodiments the host cell is a prokaryotic cell. In some embodiments the prokaryotic cell is a gram-negative bacterial cell. In some embodiments the host cell is non-pathogenic for humans. In some embodiments the cell includes a *rhlA* gene or an ortholog thereof as well as a *rhlB* gene or an ortholog thereof. The *rhlA* gene or the ortholog thereof and/or the *rhlB* gene or ortholog thereof may be under the control of a heterologous promoter. The cell may further include a *rhlC* gene or an ortholog thereof. The *rhlC* gene or the ortholog thereof may in some embodiments be under the control of a heterologous promoter. In some embodiments the host cell is cultured. A host cell is in some embodiments grown at a temperature above 30°C.

The bacterial cell may in some embodiments be selected from the group consisting of *Pseudomonas putida, Pseudomonas chlororaphis, Pseudomonas fluorescens, Pseudomonas alcaligenes, Pseudomonas aeruginosa, Pseudomonas cepacia, Pseudomonas clemancea, Pseudomonas collierea, Pseudomonas luteola, Pseudomonas stutzeri, Pseudomonas teessidea, Escherichia coli, Renibacterium salmoninarum, Cellulomonas cellulans, Tetragenococcus koreensis, Burkholderia glumae, Burkholderia mallei, Burkholderia pseudomallei, Burkholderia plantarii, Burkholderia thailandensis, Acinetobacter calcoaceticus, Enterobacter asburiae, Enterobacter hormaechei, Pantoea stewartii* and *Pantoea ananatis.*

In some embodiments the process according to the first aspect is a process for the *in situ* isolation of a biosurfactant. The process includes culturing a host cell capable of producing the biosurfactant. The process further includes allowing the formation and/or accumulation of foam that includes the biosurfactant at the culture broth-gas interface. The process also includes applying the foam directly onto an adsorbent which effects collapse of the foam. Furthermore the process includes isolating the adsorbed biosurfactant by desorption. In some embodiments the process includes one or more further steps of purifying the biosurfactant. In some embodiments of the process according to the first aspect the biosurfactant is one or more of a glycolipid, a lipopeptide, a fatty acid, a neutral lipid, a phospholipid and a polymeric surfactant. A respective glycolipid may include one or more glycolipids selected from the group consisting of a rhamnolipid, a trehalolipid, a sophorolipid and a cellobiolipid. A respective lipopeptide may include one or more lipopeptides selected from the group consisting of serrawettin, viscosin, surfactin, subtilisin, gramicidin and polymyxin. A respective polymeric surfactant may include one or more polymeric surfactants selected from the group consisting of emulsan, biodispersan, mannan-lipid-protein, liposan and carbohydrate-protein-lipid. In some embodiments the host cell is a prokaryotic cell. The prokaryotic cell is in some embodiments a gram-negative bacterial cell. In some embodiments the host cell is non-pathogenic for humans. In some embodiments the cell includes a *rhlA* gene or an ortholog thereof as well as a *rhlB* gene or an ortholog thereof. The *rhlA* gene or the ortholog thereof and/or the *rhlB* gene or ortholog thereof may in some embodiments be under the control of a heterologous promoter. The cell may further include a *rhlC* gene or an ortholog thereof. The *rhlC* gene or the ortholog thereof may in some embodiments be under the control of a heterologous promoter.

The bacterial cell may in some embodiments be selected from the group consisting of *Pseudomonas putida, Pseudomonas chlororaphis, Pseudomonas fluorescens, Pseudomonas alcaligenes, Pseudomonas aeruginosa, Pseudomonas cepacia, Pseudomonas clemancea, Pseudomonas collierea, Pseudomonas luteola, Pseudomonas stutzeri, Pseudomonas teessidea, Escherichia coli, Renibacterium salmoninarum, Cellulomonas cellulans, Tetragenococcus koreensis, Burkholderia glumae, Burkholderia mallei, Burkholderia pseudomallei, Burkholderia plantarii, Burkholderia thailandensis, Acinetobacter calcoaceticus, Enterobacter asburiae, Enterobacter hormaechei, Pantoea stewartii* and *Pantoea ananatis.*

In some embodiments of the process according to the first aspect the biosurfactant is one or more of a glycolipid, a lipopeptide, a fatty acid, a neutral lipid, a phospholipid and a polymeric surfactant. The glycolipid may include one or more glycolipids selected from the group consisting of a rhamnolipid, a trehalolipid, a sophorolipid and a cellobiolipid. The lipopeptide may include one or more lipopeptides selected from the group consisting of serrawettin, viscosin, surfactin, subtilisin, gramicidin and polymyxin. The polymeric surfactant may include one or more polymeric surfactants selected from the group consisting of emulsan, biodispersan, mannan-lipid-protein, liposan and carbohydrate-protein-lipid.

In some embodiments of the process according to the first aspect the foam formed and/or accumulated at the culture broth-gas interface is non-collapsed. In some embodiments the foam includes bubbles, micelles, spherical micelles, cylindrical micelles, and/or vesicles. In some embodiments the foam includes a lamellar or lamellar-like structure.

In some embodiments of the process according to the first aspect the adsorbent is adsorbent material in bulk.

In some embodiments of the process according to the first aspect the adsorbent is a polystyrene resin. Such a polystyrene resin may be cross-linked with divinylbenzene. A respective polystyrene resin may also be not-cross linked with divinylbenzene. In some embodiments of the process according to the third aspect the adsorbent is activated carbon.

In some embodiments the adsorbent is silicate or aluminum hydroxide.

In a second aspect the invention provides a process for the isolation of a hydrophobic compound from a hydrophilic medium. The process includes allowing the formation and/or accumulation of foam that includes the hydrophobic compound at the hydrophilic medium-gas interface. The process also includes applying the foam directly onto an adsorbent, which is in the form of particles and effects collapse of the foam. The process further includes isolating the adsorbed hydrophobic compound by desorption. In said process, the adsorbent is hydrophobic

In some embodiments of the process according to second aspect consists of the steps defined above. In such an embodiment the process consists of (a) allowing the formation and/or accumulation of foam that includes the hydrophobic compound at the hydrophilic medium-gas interface; (b) applying the foam directly onto an adsorbent, which effects collapse of the foam; and (c) isolating the adsorbed hydrophobic compound by desorption.

In some embodiments the process according to the second aspect further includes one or more further steps of purifying the hydrophobic compound.

In some embodiments the process according to the second aspect consists of (a) allowing the formation and/or accumulation of foam that includes the hydrophobic compound at the hydrophilic medium-gas interface, respectively; (b) applying the foam directly onto an adsorbent, which effects collapse of the foam; (c) isolating the adsorbed hydrophobic compound by desorption; and (d) one or more steps of further purifying the hydrophobic compound.

In some embodiments of the process according to the second aspect the foam formed and/or accumulated at the culture broth-gas interface is non-collapsed. In some embodiments the foam includes bubbles, micelles, spherical micelles, cylindrical micelles, and/or vesicles. In some embodiments the foam includes a lamellar or lamellar-like structure.

In some embodiments of the process according to the second aspect the adsorbent is adsorbent material in bulk.

In some embodiments of the process according to the second aspect the adsorbent is a polystyrene resin. Such a polystyrene resin may be cross-linked with divinylbenzene. A respective polystyrene resin may also be not-cross linked with divinylbenzene. In some embodiments of the process according to the third aspect the adsorbent is activated carbon.

In some embodiments of the process according to the second aspect the adsorbent is hydrophilic if the solvent of the culture broth is hydrophilic. In some embodiments the adsorbent is silicate or aluminum hydroxide.

According to a third aspect, the present invention provides a process for the isolation of a hydrophilic compound from a hydrophobic medium. The process includes allowing the formation and/or accumulation of foam that includes the hydrophilic compound at the hydrophobic medium-gas interface. Further the process includes applying the foam directly onto an adsorbent which is in the form of particles and effects collapse of the foam. The process also includes isolating the adsorbed hydrophilic compound by desorption. In said process, the adsorbent is hydrophilic.

In some embodiments of the process according to third aspect consists of the steps defined above. In such an embodiment the process consists of (a) allowing the formation and/or accumulation of foam that includes the hydrophilic compound at the hydrophobic medium-gas interface; (b) applying the foam directly onto an adsorbent, which effects collapse of the foam; and (c) isolating the adsorbed hydrophilic compound by desorption.

In some embodiments the process according to the third aspect further includes one or more further steps of purifying the hydrophilic compound.

In some embodiments the process according to the third aspect consists of (a) allowing the formation and/or accumulation of foam that includes the hydrophilic compound at the hydrophobic medium-gas interface; (b) applying the foam directly onto an adsorbent, which effects collapse of the foam; (c) isolating the adsorbed hydrophilic compound by desorption; and (d) one or more steps of further purifying the hydrophilic compound.

In some embodiments of the process according to the third aspect the foam formed and/or accumulated at the culture broth-gas interface is non-collapsed. In some embodiments the foam includes bubbles, micelles, spherical micelles, cylindrical micelles, and/or vesicles. In some embodiments the foam includes a lamellar or lamellar-like structure.

In some embodiments of the process according to the third aspect the adsorbent is adsorbent material in bulk.

In some embodiments of the process according to the third aspect the adsorbent is a polystyrene resin. Such a polystyrene resin may be cross-linked with divinylbenzene. A respective polystyrene resin may also be not-cross linked with divinylbenzene. In some embodiments of the process according to the third aspect the adsorbent is activated carbon.

In some embodiments of the process according to the third aspect the adsorbent is hydrophilic if the solvent of the culture broth is hydrophilic. In some embodiments the adsorbent is silicate or aluminum hydroxide.

According to a fourth aspect, the present disclosure provides a biosurfactant. The biosurfactant is obtainable by a process as described above.

According to a fifth aspect, the present disclosure provides for the use of a host cell for producing a biosurfactant. The host cell is capable of expressing the biosurfactant. In the use the host cell is being cultured and allowed to express the biosurfactant.

The host cell is in some embodiments a prokaryotic cell. In some embodiments the prokaryotic cell is a gram-negative bacterial cell. In some embodiments the host cell is non-pathogenic for humans. In some embodiments the cell includes a *rhlA* gene or an ortholog thereof as well as a *rhlB* gene or an ortholog thereof. The *rhlA* gene or the ortholog thereof and/or the *rhlB* gene or ortholog thereof may be under the control of a heterologous promoter. The cell may further include a *rhlC* gene or an ortholog thereof. The *rhlC* gene or the ortholog thereof may in some embodiments be under the control of a heterologous promoter. The bacterial cell may in some embodiments be selected from the group consisting of *Pseudomonas putida, Pseudomonas chlororaphis, Pseudomonas fluorescens, Pseudomonas alcaligenes, Pseudomonas aeruginosa, Pseudomonas cepacia, Pseudomonas clemancea, Pseudomonas collierea, Pseudomonas luteola, Pseudomonas stutzeri, Pseudomonas teessidea, Escherichia coli, Renibacterium salmoninarum, Cellulomonas cellulans, Tetragenococcus koreensis, Burkholderia glumae, Burkholderia mallei, Burkholderia pseudomallei, Burkholderia plantarii, Burkholderia thailandensis, Acinetobacter calcoaceticus, Enferobacter asburiae, Enterobacter hormaechei, Pantoea stewartii* and *Pantoea ananatis.* In some embodiments the host cell is cultured. The host cell may in some embodiments be grown at a temperature above 30°C.

In some embodiments of the use according to the fifth aspect the biosurfactant is one or more of a glycolipid, a lipopeptide, a fatty acid, a neutral lipid, a phospholipid and a polymeric surfactant. The glycolipid may include one or more glycolipids selected from the group consisting of a rhamnolipid, a trehalolipid, a sophorolipid and a cellobiolipid. The lipopeptide may include one or more lipopeptides selected from the group consisting of serrawettin, viscosin, surfactin, subtilisin, gramicidin and polymyxin. The polymeric surfactant may include one or more polymeric surfactants selected from the group consisting of emulsan, biodispersan, mannan-lipid-protein, liposan and carbohydrate-protein-lipid.

In some embodiments of the use according to the fifth aspect the foam formed and/or accumulated at the culture broth-gas interface is non-collapsed. In some embodiments the foam includes bubbles, micelles, spherical micelles, cylindrical micelles, and/or vesicles. In some embodiments the foam includes a lamellar or lamellar-like structure.

In some embodiments of the use according to the fifth aspect the amphipathic compound is produced by culturing a host cell capable of producing the biosurfactant. In some embodiments the host cell is cultured. A host cell is in some embodiments grown at a temperature above 30°C.

It is to be noted that as used in this specification, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein. Likewise reference to "a cell" includes a single cell as well as a plurality of cells.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. Thus the terms "comprising", "including," containing", "having" etc. shall be read expansively or open-ended and without limitation. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms, albeit the terms have different meanings as explained above.

It is furthermore understood that slight variations above and below a stated range can be used to achieve substantially the same results as a value within the range. Also, unless indicated otherwise, the disclosure of ranges is intended as a continuous range including every value between the minimum and maximum values.

### DETAILED DESCRIPTION OF THE INVENTION

With the aim of optimizing the isolation and/or purification, i.e., recovery of biosurfactants by continuous separation from production medium (culture broth) the inventors aimed at providing an easy-to-handle, efficient, low energy and time consuming and thus cost-conscious and sustainable process for the *in situ* isolation of a biosurfactant during its production. Put differently, by simplifying the thus-far known components and steps required for such a production process and by deviating from the usual practice in the fermentation of biosurfactant producing microorganisms, the inventors could succeed in the provision of an economic process on a large scale. Indeed, when the inventors treaded an unconventional path in the *in situ* separation of foam that included a desired biosurfactant, they observed to their surprise that their uncommon set-up of the production process allows an easy-to-handle, efficient, low energy and time consuming and thus cost-conscious and sustainable process for the *in situ* isolation of a biosurfactant during its production.

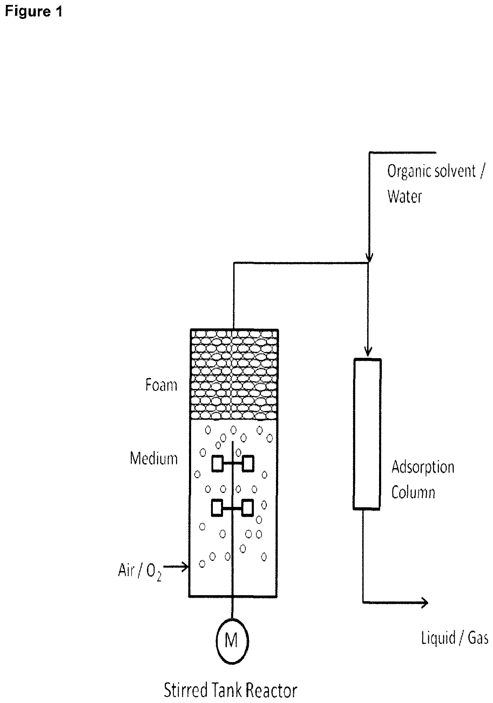

Specifically, rather than separating culture broth from the produced biosurfactant by centrifugation or filtration or the like in order to collect cell-free foam, rather than embedding biosurfactant producing microorganisms in magnetic particles in order to retain them when separating the foam from the culture broth, and rather than collapsing the foam either mechanically or chemically, the inventors explicitly allow, even support the formation and/or accumulation of foam comprising a desired biosurfactant such that the fermenter "overflows" with the aim of directly applying the foam onto an adsorbent which effects collapse of the foam. Thus, no sophisticated techniques such as magnetic particles and high-gradient magnetic separation as in Heyd et al. (2011) or a device called "flounder" as applied in Glazyrina et al. (2008) that do in fact restrict an industrial-size scale of a biosurfactant fermentation process and render a process complicated are required (see Fig. 1).

Indeed, while those in the art aimed at collecting cell-free foam comprising a desired biosurfactant that is afterwards either mechanically or chemically collapsed, the process of the present invention does not require to separate biosurfactant producing cells, but exploits the foam collapsing property of the adsorbent, either a hydrophobic or hydrophilic material, that is preferably in the form of particles, and that is preferably in bulk. Accordingly, the adsorbent therefore provides a large surface which effects collapse of foam, which includes the desired biosurfactant. Also, the adsorbent allows flow-through of biosurfactant producing microorganisms and/or allows washing off these microorganisms, just in case they have a tendency to adsorb at the adsorbent material.

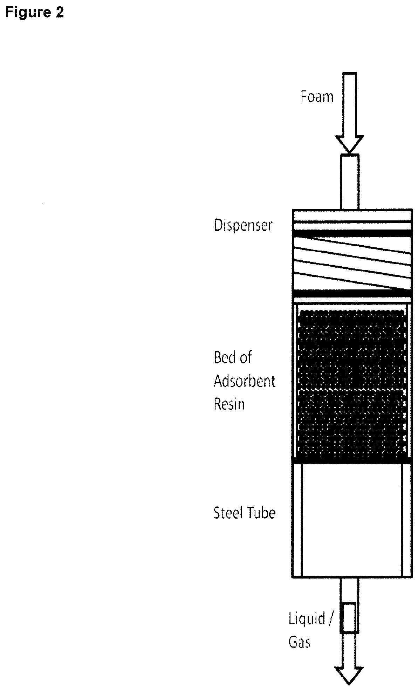

However, the inventive principle elucidated by the present inventors is not only applicable for the isolation of biosurfactants. Rather, it can plausibly be generalized to the isolation of amphipathic compounds from a hydrophobic and/or hydrophilic medium. Likewise, the inventive principle is plausibly generalizable to the isolation of a hydrophobic compound from a hydrophilic medium or the isolation of a hydrophilic compound from a hydrophobic medium. This is so because, the inventive principle is based on the observation that foam that includes the desired compound that is allowed to form and/or accumulate at the medium-gas interface is directly applied onto an adsorbent which then effects collapse of the respective foam. Thus, the decisive step is the direct application of the foam comprising the desired compound onto an adsorbent (see Example 1 and Figures 1 and 2) which unifies the makes the methods of the present invention and makes them distinguishable from prior art methods, too.

Hence, in a first aspect the present invention provides a method of isolating an amphipathic compound from a hydrophobic or hydrophilic medium. The method includes
- allowing the formation and/or accumulation of foam comprising the amphipathic compound at the hydrophobic or hydrophilic medium-gas interface, respectively,
- applying the foam directly onto an adsorbent which effects collapse of the foam, and
- isolating the adsorbed amphipathic compound by desorption,
wherein said adsorbent is hydrophobic if the solvent of the culture broth is hydrophilic,
wherein said adsorbent is hydrophilic if the solvent of the culture broth is hydrophobic,
wherein the adsorbent is in the form of particles.
Optionally the method includes one or more further steps of purifying the amphipathic compound.

In a second aspect the present invention provides a method of isolating a hydrophobic compound from a hydrophilic medium. The method includes
- allowing the formation and/or accumulation of foam comprising the hydrophobic compound at the hydrophilic medium-gas interface,
- applying the foam directly onto an adsorbent which effects collapse of the foam, and
- isolating the adsorbed hydrophobic compound by desorption,
wherein said adsorbent is hydrophobic and the adsorbent is in the form of particles. Optionally the method also includes one or more further steps of purifying the hydrophobic compound.

In a third aspect the present invention provides a method of isolating a hydrophilic compound from a hydrophobic medium. The method includes
- allowing the formation and/or accumulation of foam comprising the hydrophilic compound at the hydrophobic medium-gas interface,
- applying the foam directly onto an adsorbent which effects collapse of the foam, and
- isolating the adsorbed hydrophilic compound by desorption,
wherein said adsorbent is hydrophilic and the adsorbent is in the form of particles. Optionally the method further includes one or more further steps of purifying the hydrophilic compound.

The methods of the present invention can, for example, be applied to isolate organic waste from water, to remove one or more surface active contaminants from waste water streams, to enrich one or more biosurfactants, to remove one or more contaminants from crude oil, etc.

In a preferred embodiment of the method of the first aspect of the present invention, the amphipathic compound is a biosurfactant. The biosurfactant is preferably produced by culturing a host cell that is capable of producing the biosurfactant.

Hence, in a preferred aspect the present invention provides a method for the *in situ* isolation of a biosurfactant. The method includes
(a) culturing a cell capable of producing the biosurfactant,
(b) allowing the formation and/or accumulation of foam comprising the biosurfactant at the culture broth-gas interface,
(c) applying the foam directly onto an adsorbent which effects collapse of the foam, and
(d) isolating the adsorbed biosurfactant by desorption.
Optionally the method further includes one or more further steps for purifying the biosurfactant. In some embodiments the method consists of
(a) culturing a cell capable of producing the biosurfactant,
(b) allowing the formation and/or accumulation of foam comprising the biosurfactant at the culture broth-gas interface,
(c) applying the foam directly onto an adsorbent which effects collapse of the foam, and
(d) isolating the adsorbed biosurfactant by desorption.
In some embodiments the method consists of (a) culturing a cell capable of producing the biosurfactant; (b) allowing the formation and/or accumulation of foam comprising the biosurfactant at the culture broth-gas interface; (c) applying the foam directly onto an adsorbent which effects collapse of the foam; (d) isolating the adsorbed biosurfactant by desorption; and (e) one or more further steps for purifying the biosurfactant.

As it is known that product inhibition might occur at higher biosurfactant concentration, continuous separation of a biosurfactant from culture broth is desirable for continuous biosurfactant production with a permanent product removal to achieve higher product yields. Accordingly, the method of the present invention preferably allows continuous separation of a biosurfactant from culture broth. This means that a biosurfactant can be removed/separated from the fermentation process while fermentation (culturing of biosurfactant producing cells) is continued.

The overall concept of the preferred method of the present invention is the *in situ* isolation of a biosurfactant. *"In situ"* when used herein means that the isolation of a biosurfactant takes place (occurs/is done) in place, i.e., it is, after and/or during its production (in) a reactor system, preferably an integrated reactor system, isolated. The *"in situ"* isolation thus preferably includes culturing of cells capable of producing a biosurfactant, allowing the formation and/or accumulation of foam that includes the biosurfactant at the culture broth-gas interface, applying the foam directly onto an absorbent which effects collapse of the foam and isolating the biosurfactant released from the foam and being thus adsorbed at the adsorbent by desorption. Advantageously, any biosurfactant producing cells which may be contained in the foam that is collapsed at the adsorbent can be fed back into the reactor in order to continue the production of the biosurfactant.

The term "medium" when used herein includes any liquid medium which is either hydrophilic or hydrophobic. It may also be a medium that is both hydrophilic and hydrophobic, i.e., a mixture. The medium preferably contains a solvent. A preferred medium is a culture broth.

Hydrophilic ("water-loving") matter, including surfaces and liquids, also termed lipophobic ("fat-fearing"), contains molecules which can form dipole-dipole interactions with water molecules. Hydrophilic liquids thus dissolve therein. Hydrophobic ("water-fearing") matter has a tendency to separate from water. A related term is the indication lipophilic ("fat-loving"). Lipophilic matter attracts non-polar organic compounds, such as oils, fats, or greases. It is understood that the terms "hydrophobic" and "lipophilic" are not in all cases synonymous. For example, perfluorocarbon compounds are both hydrophobic and oleophobic, i.e. lack an affinity for oils. Such compounds accordingly have a tendency to separate from both water and hydrocarbons (though the latter to a lesser extent than from water). Examples of a hydrophilic liquid include, but are not limited to water, acetone, methanol, ethanol, propanol, isopropanol, butanol, tetrahydrofuran, pyridine, chloroform, ethylene glycol monobutyl ether, pyridine, ethyl acetate, acetonitrile, dimethylformamide, N,N-dimethyl acetamide, N-methylpyrrolidone, formic acid, formamide, and a polar ionic liquid. Examples of a polar ionic liquid include, but are not limited to, 1-ethyl-3-methylimidazolium tetrafluoroborate, N-butyl-4-methylpyridinium tetrafluoroborate, 1,3-dialkylimidazolium-tetrafluoroborate, 1,3-dialkylimidazolium-hexafluoroborate, 1-ethyl-3-methylimidazolium bis(pentafluoroethyl)phosphinate, 1-butyl-3-methylimidazolium tetra-kis(3,5-bis(trifluoromethylphenyl)borate, tetrabutyl-ammonium bis(trifluoromethyl)imide, ethyl-3-methylimidazolium trifluoromethanesulfonate, 1-butyl-3-methylimidazolium methylsulfate, 1-n-butyl-3-methylimidazolium ([bmim]) octylsulfate, and 1-n-butyl-3-methylimidazolium tetrafluoroborate. Examples of a non-polar liquid include, but are not limited to mineral oil, hexane, heptane, cyclohexane, benzene, toluene, dichloromethane, chloroform, carbon tetrachloride, carbon disulfide, dioxane, diethyl ether, diisopropylether, methyl propyl ketone, methyl isoamyl ketone, methyl isobutyl ketone, cyclohexanone, isobutyl isobutyrate, ethylene glycol diacetate, and a non-polar ionic liquid. Examples of a non-polar ionic liquid include, but are not limited to, 1-ethyl-3-methylimidazolium bis[(trifluoromethyl)sulfonyl]amide bis(triflyl)amide, 1-ethyl-3-methylimidazolium bis[(trifluoromethyl)sulfonyl]amide trifluoroacetate, 1-butyl-3-methylimidazolium hexafluorophosphate, 1-hexyl-3-methylimidazolium bis-(trifluoromethylsulfonyl)imide, 1-butyl-3-methylimidazolium bis(trifluoromethylsulfonyl)imide, trihexyl(tetradecyl)phosphonium bis[oxalato(2-)]borate, 1-hexyl-3-methyl imidazolium tris-(pentafluoroethyl)trifluorophosphate, 1-butyl-3-methyl-imidazolium hexafluorophosphate, tris-(pentafluoroethyl)trifluorophosphate, trihexyl(tetradecyl)phosphonium, N"-ethyl-N,N,N',N'-tetramethylguanidinium, 1-butyl-1-methyl pyrrolidinium tris(pentafluoroethyl) trifluoro-phosphate, 1-butyl-1-methyl pyrrolidinium bis(trifluoromethylsulfonyl) imide, 1-butyl-3-methyl imidazolium hexafluorophosphate, 1-ethyl-3-methylimidazolium bis(trifluoromethylsulfonyl)-imide and 1-n-butyl-3-methylimidazolium.

The surface energy as defined by Thomas Young in 1805 is the interaction between forces of cohesion and forces of adhesion, thereby determining whether wetting or spreading of a liquid droplet on a surface occur. Surface energy can also be taken as the work required to increase the surface of a material by a defined area. Thus surface energy indicates the disruption of chemical bonds upon creation of a surface. Surfaces have to be less energetically favourable than a bulk phase, since otherwise surface energy would generate surfaces. Adhesion of matter is favoured if the free energy of adhesion at a given surface is negative. Generally, molecules at a surface tend to reduce free energy by interacting with other matter. Matter with the smallest difference of surface energy tends to undergo interactions with each other rather than with matter with a larger difference of surface energy, as such interaction is thermodynamically most stable. Accordingly, surfaces of matter of comparable surface energy attract each other. An illustrative example of a liquid is water, which is matter with high surface energy. While interactions of surfaces with liquids can generally be described using surface energy, interactions of other surfaces with water are typically described in terms of hydrophobicity, as water is one of most hydrophilic materials. Hydrophobicity generally decreases with increasing surface energy. As an illustrative example, a hydrophilic surface such as glass has relatively high surface energy, while a hydrophobic surface such as a fluoropolymer (e.g. polytetrafluoroethylene) has a relatively low surface energy.

An amphiphilic, also called amphiphatic, compound has both hydrophilic and hydrophobic properties. Generally an amphiphilic compound has certain moieties that are hydrophilic and certain moieties that are hydrophobic. Typically an amphiphilic compound is a surfactant. A surfactant is generally an amphipathic organic compound, such as an anionic, cationic, zwitterionic, or nonionic compound. The surfactant may for instance be a hydrocarbon compound, a hydroperfluoro carbon compound or a perfluorocarbon compound.

"Applying the foam directly onto an adsorbent" includes removing the foam and/or skimming off the foam from the surface of the culture broth and/or transferring it to an adsorbent material. Accordingly, this step advantageously includes the transfer of the foam onto an adsorbent material, preferably one as described herein. The transfer of the foam is preferably done by a foam fractionation column or a pipe that is preferably installed on top of the reactor, for example, installed instead of the exhaust air cooler. Accordingly, foam is pressed out of the reactor via the foam fractionation column or pipe and preferably directly guided to adsorbent material.

Thus, preferably at no stage during the direct application of the foam onto an adsorbent is foam collected in a vessel, container or receptacle or the like in order to collapse it, for example, mechanically or chemically. Rather, the foam is directly brought into contact with (applied onto) an adsorbent which effects collapse of the foam. The direct application of the foam onto an adsorbent is the key difference to prior art approaches undertaken in the continuous separation of a hydrophobic compound from a hydrophilic medium, a hydrophilic compound from a hydrophobic medium, or an amphipathic compound such as a biosurfactant from a hydrophobic and/or hydrophilic medium by foam fractionation. In fact, as mentioned herein, those of skill in the art collected the foam and collapsed it either mechanically or chemically, once they have fractionated it from the culture broth. However, by taking a rather unconventional approach, the present inventors applied foam comprising a desired hydrophobic, hydrophilic or amphipathic compound, in particular a biosurfactant directly onto an adsorbent and have surprisingly found that the adsorbent effects collapse of the foam, thereby releasing the desired compound, preferably a biosurfactant that gets adsorbed by the adsorbent material.

Accordingly, the method of the present invention does in general preferably exclude collecting, transferring or the like, the foam into a container, vessel, receptacle or the like with the aim of collapsing it. Also, the method of the present invention does in general preferably exclude chemically, e.g., by defoaming through, e.g., a chemical such as citric acid or an anti-foaming agent, or mechanically, e.g., by stirring or freezing, collapsing the foam.

The adsorbent is preferably adsorbent material in bulk such as loose bulk or unpackaged bulk. In bulk, the adsorbent provides advantageously a large surface which facilitates collapse of the foam. It is thus also preferred that the adsorbent is in the form of particles. The bulk form of the adsorbent does not only provide a large surface, it also offers the advantage that the adsorbent is not tightly packaged such as a tightly packaged adsorbent column. Accordingly, because of its loose packaging the adsorbent has sufficient adsorbing capacity and/or the capability to let air or gas, culture medium and/or cells from the culture broth pass through. Likewise, absorbent material in bulk allows washing off cells which may have a tendency to adsorb at (onto) the adsorbent material. Hence, cells can be fed back into the reactor.

For example, the adsorbent is hydrophobic if the solvent of the culture broth is hydrophilic. By the same token, the adsorbent is hydrophilic if the solvent of the culture broth is hydrophobic.

However, the adsorbent which can either be hydrophobic or hydrophilic, dependent on the solvent of the culture broth, does preferably not adsorb compounds other than the desired compound, preferably the adsorbent does not adsorb cells producing the biosurfactant. Yet, even if the adsorbent would adsorb cells, the skilled person can readily apply a washing step with a solvent that does not desorb the biosurfactant from the adsorbent, but the cells.

Accordingly, the method of the present invention preferably includes a washing step prior to isolating the adsorbed compound, in particular a biosurfactant, by desorption from the adsorbent. The cells that are flushed (washed off) can preferably be fed back into the reactor, more specifically into the culture broth, where they can again produce the biosurfactant. Thus, the preferred method of the present invention also includes the step (after the washing step) of feeding back host cells flushed off the adsorbent into the culture broth.

A preferred hydrophobic adsorbent is a polystyrene resin, preferably cross-linked with divinylbenzene or not cross-linked with divinylbenzene, or activated carbon.

A preferred hydrophilic adsorbent is silicate or aluminium hydroxide.

The term "culturing" cells or "cultivation of cells" refers to the seeding of the cells into a culture vessel, to the feeding of the cells into the culture broth of the reactor, growing of the cells in culture broth in the logarithmic phase and/or maintaining the growth of cells in culture broth. Culturing can be done in any container suitable for culturing cells, for instance in dishes, roller bottles or in bioreactors such as the WAVE bioreactor system, by using batch, fed-batch, continuous systems, hollow fiber, and the like.

The term "isolating" or "isolation" in all its grammatical forms when used in the context of the methods of the present invention indicates that a compound has been removed from its original environment, e.g. its normal physiological environment, such as a natural source, or that a peptide or nucleic acid is synthesized. An isolated cell or isolated cells may for instance be included in a different medium such as an aqueous solution than provided originally, or placed in a different physiological environment. Typically isolated cells, peptides or nucleic acid molecule(s) constitute a higher fraction of the total cells, peptides or nucleic acid molecule(s) present in their environment, e.g. solution/suspension as applicable, than in the environment from which they were taken. By "isolated" in reference to a hydrophobic compound, a hydrophilic compound and/or an amphipathic compound is meant a corresponding compound that is isolated from a natural source or that is synthesized. The term "isolated" in this context includes that a hydrophobic compound, a hydrophilic compound and/or an amphipathic compound is obtained, harvested, recovered, isolated, achieved, received, and/or gained from a medium. In case of the isolation of a hydrophobic compound in accordance with the teaching of the present invention the medium is hydrophilic. If the compound to be isolated is hydrophilic, the medium is hydrophobic. In case of the isolation of an amphipathic compound in accordance with the teaching of the present invention, the medium is hydrophobic or hydrophilic. Accordingly, the methods of the present invention could equally be methods for recovering, producing, harvesting, etc. a desired compound as described herein from a medium. In a preferred aspect, the amphipathic compound, in particular a biosurfactant is isolated from culture broth containing a host cell capable of producing the biosurfactant and/or is isolated from said host cell.

"Allowing the formation of foam" or "allowing foaming" as used herein means that foam comprising the desired compound as taught herein is allowed to be formed in an amount such that it is above the critical micelle concentration (CMC) in the medium, where it can form foam at the medium-gas interface (e.g., at the surface of a culture broth). In order to support or even enhance foam formation preferably air bubbles are introduced into the medium, e.g., into the culture broth where a host cell is cultured that produces a desired compound such as a biosurfactant. These bubbles, as they rise to the surface, pull out the desired compound from the bulk to the top of the medium because of its either hydrophobic or hydrophilic character, whereby the medium has the opposite character (i.e., the medium is hydrophilic if the compound is hydrophobic or the medium is hydrophobic if the compound is hydrophilic), thereby creating foam and, thus, bringing down the concentration of the desired compound in the medium to below CMC. The CMC can be determined as described in Dominguez et al. (1997), J Chem Education 74 (10):1227-1231. The adhered desired compound stabilizes the gas bubbles so that they form foam.

In a preferred embodiment of the first aspect of the present invention, "allowing the formation of foam" or "allowing foaming" as used herein means that the host cell producing a desired biosurfactant is allowed to produce the biosurfactant in an amount such that it is above the critical micelle concentration (CMC) in the culture broth.

"Allowing the accumulation of foam" as used herein means that once foam is formed, it is allowed to enrich or increase at the medium (e.g. culture broth)-gas interface (e.g., surface of the culture broth). Accordingly, once it has accumulated, it is applied directly onto an adsorbent material as described herein. Advantageously, the foam is pressed out of the reactor due to the continuous air/gas supply via a foam fractionation column or pipe and directly guided to adsorbent material.

Given the fact that the method of the present invention is designed to allow the formation of foam and/or accumulation of foam at the medium-gas interface, it is preferred that the foam formed and/or accumulated at the medium-gas interface is non-collapsed.

Accordingly, it is likewise preferred that the foam includes bubbles, micelles, spherical micelles, cylindrical micelles, and/or or vesicles such as unilamellar or bilamellar vesicles.

Also, it is preferred that the foam includes a lamellar or lamellar-like structure, for example, composed of dense micelles in various forms such as spherical micelles, globular micelles, cylindrical micelles, and/or vesicles such as unilamellar or bilamellar vesicles and the like.

The "isolation" step of the method of the present invention also includes that the amphipathic, hydrophobic or hydrophilic compound is to be released from the adsorbent, for example, by desorption with a solvent in which the compound, which is to be isolated, is soluble. Typical desorption agents for a biosurfactant include methanol, isopropanol, MTBE or ethylacetate or the like.

The isolation step may optionally be followed by one or more steps of purifying the compound. For example, the compound may be subject to further chromatography.

Chromatography may for example be carried out in the form of a liquid chromatography such as capillary electrochromatography, HPLC (high performance liquid chromatography) or UPLC (ultrahigh pressure liquid chromatography) or as a gas chromatography. The chromatography technique may be a process of column chromatography, of batch chromatography, of centrifugal chromatography or a method of expanded bed chromatography, as well as electrochromatographic, electrokinetic chromatography. It may be based on any underlying separation technique, such as adsorption chromatography, hydrophobic interaction chromatography or hydrophobic charge induction chromatography, size exclusion chromatography (also termed gel-filtration), ion exchange chromatography or affinity chromatography and may also be a method of capillary gas chromatography. Another example of purification is an electrophoretic technique, such as preparative capillary electrophoresis including isoelectric focusing. Examples of electrophoretic methods are for instance free flow electrophoresis (FFE), polyacrylamide gel electrophoresis (PAGE), capillary zone or capillary gel electrophoresis. An isolation may include may include the combination of similar methods. Also, a purification step may include filtration.

In some embodiments the amphipathic compound is a biosurfactant. Biosurfactants (sometimes abbreviated herein as "BS") are amphiphilic compounds produced by living organisms, in particular microorganisms including bacteria, fungi and yeasts.Biosurfactants are preferably produced on living surfaces, mostly microbial cell surfaces, or excreted extracellularly and contain hydrophobic and hydrophilic moieties that reduce surface tension (ST) and interfacial tensions between individual molecules at the surface and interface, respectively. Biosurfactants exhibit emulsification properties. A biosurfactant may, for example, have one of the following structures: mycolic acid, glycolipids, polysaccharide-lipid complex, lipoprotein or lipopeptide, phospholipid, or the microbial cell surface itself.

In a preferred embodiment of the present invention, the biosurfactant is one or more selected from the group consisting of glycolipids, lipopeptides, fatty acids, neutral lipids, phospholipids and polymeric surfactants.

Glycolipids are the most common types of biosurfactants. The constituent mono-, di-, tri- and tetrasaccharides typically include glucose, mannose, galactose, glucuronic acid, rhamnose, and galactose sulphate. The fatty acid component usually has a composition similar to that of the phospholipids of the same microorganism. The glycolipids can be categorized as trehalose lipids, sophorolipids, rhamnolipids, cellobiolipids. Accordingly, the glycolipid is preferably one or more glycolipid selected from the group consisting of rhamnolipids, trehalolipids, sophorolipids and cellobiolipids.

The fatty acids produced from alkanes by microbial oxidations have received maximum attention as surfactants. Besides the straight-chain acids, microorganisms produce complex fatty acids containing OH groups and alkyl branches. Some of these complex acids, for example corynomucolic acids, are surfactants.

Phospholipids are major components of microbial membranes. When certain CₓH_{y}-degrading bacteria or yeast are grown on alkane substrates, the level of phospholipids increases greatly. Phospholipids from hexadecane-grown *Acinetobacter* sp. have potent surfactant properties. Phospholipids produced by *Thiobacillus thiooxidans* have been reported to be responsible for wetting elemental sulphur, which is necessary for growth.

The lipopeptide is preferably one or more lipopeptide selected from the group consisting of serrawettin, viscosin, surfactin, subtilisin, gramicidin and polymyxin.

Gramicidin S: Many bacteria produce a cyclosymmetric decapeptide antibiotic, gramicidin S. Spore preparations of *Brevibacterium brevis* contain large amounts of gramicidin S bound strongly to the outer surface of the spores. Mutants lacking gramicidin S germinate rapidly and do not have a lipophilic surface. The antibacterial activity of gramicidin S is due to its high surface activity.

Polymixins: These are a group of antibiotics produced by *Brevibacterium polymyxa* and related bacilli. Polymixin B is a decapeptide in which amino acids 3 through 10 form a cyclic octapeptide. A branched chain fatty acid is connected to the terminal 2,4-diaminobutyric acid (DAB). Polymixins are able to solubilize certain membrane enzymes.

Surfactin (subtilysin): One of the most active biosurfactants produced by *B. subtilis* is a cyclic lipopeptide surfactin. The yield of surfactin produced by *B. subtilis* can be improved to around 0.8 g/l by continuously removing the surfactant by foam fractionation and addition of either iron or manganese salts to the growth medium.

Most of polymeric microbial surfactants are polymeric heterosaccharide containing proteins. *Acinetobacter calcoaceticus RAG-1 (ATCC 31012) emulsan*: A bacterium, RAG-1, was isolated during an investigation of a factor that limited the degradation of crude oil in sea water. This bacterium efficiently emulsified C*ₓ*H*_{y}* in water. This bacterium, *Acinetobacter calcoaceticus,* was later successfully used to clear a cargo compartment of an oil tanker during its ballast voyage. The cleaning phenomenon was due to the production of an extracellular, high molecular weight emulsifying factor, emulsan.

The polysaccharide protein complex of Acinetobacter calcoaceticus BD413: A mutant of *A. calcoaceticus* BD4, excreted large amounts of polysaccharide together with proteins. The emulsifying activity required the presence of both polysaccharide and proteins.

Other Acinetobacter emulsifiers: Extracellular emulsifier production is widespread in the genus Acinetobacter. In one survey, 8 to 16 strains of *A. calcaoceticus* produced high amounts of emulsifier following growth on ethanol medium. This extracellular fraction was extremely active in breaking (de-emulsifying) kerosene/ water emulsion stabilized by a mixture of Tween 60 and Span 60.

Polysaccharide-lipid complexes from yeast: The partially purified emulsifier, liposan, was reported to contain about 95% carbohydrate and 5% protein. A CₓH_{y}-degrading yeast, *Endomycopsis lipolytica* YM, produced an unstable alkane-solubilizing factor. *Torulopsis petrophilum* produced different types of surfactants depending on the growth medium. On water-insoluble substrates, the yeast produced glycolipids which were incapable of stabilizing emulsions. When glucose was the substrate, the yeast produced a potent emulsifier.

Emulsifying protein (PA) from *Pseudomonas aeruginosa*: The bacterium P. *aeruginosa* has been observed to excrete a protein emulsifier. This protein PA is produced from long-chain *n*-alkanes, 1-hexadecane, and acetyl alcohol substrates; but not from glucose, glycerol or palmitic acid. The protein has a MW of 14,000 Da and is rich in serine and threonine.

Surfactants from Pseudomonas PG-1: *Pseudomonas* PG-1 is an extremely efficient hydrocarbon-solubilizing bacterium. It utilizes a wide range of CₓH_{y} including gaseous volatile and liquid alkanes, alkenes, pristane, and alkyl benzenes.

Bioflocculant and emulcyan from the filamentous *Cyanobacterium phormidium J-1*: The change in cell surface hydrophobicity of *Cyanobacterium phormidium* was correlated with the production of an emulsifying agent, emulcyan. The partially purified emulcyan has a MW greater than 10,000 Da and contains carbohydrate, protein and fatty acid esters.

The polymeric surfactant is preferably one or more polymeric surfactant selected from the group consisting of emulsan, biodispersan, mannan-lipid-protein, liposan and carbohydrate-protein-lipid.

Other preferred biosurfactants are listed in Tables 1 and 2 below:

**Table 1: Structural types of microbial surfactants**

| Surfactant | Source |
|---|---|
| Trehalose dimycolates | *Mycobacterium* sp. |
| Trehalose dicorynemycolates | *Nocardia* sp., *Rhodococcus* sp. |
| | *Arthrobacter* sp. |
| | *Corynebacterium* sp. |
| Rhamnolipids | *Pseudomonas* sp. |
| Sophorolipids Aminoacid-lipids | *Torulopsis* sp. |
| Lipopeptides | *Bacillus* sp., *Streptomyces* sp., |
| | *Corynebacterium* sp. |
| | *Mycobacterium* sp. |
| Ornithine-lipid | *Pseudomonas* sp., *Thiobacillus* sp. |
| | *Agrobacterium* sp., |
| | *Gluconobacter* sp. |
| Phosphlipids | *Candida* sp., *Corynebacterium* sp. |
| | *Micrococcus* sp. |
| | *Thiobacillus* sp. |
| Fatty acids / natural lipids | *Acinetobacter* sp., *Pseudomonas* sp. |
| | *Micrococcus* sp., *Mycococcus* sp. |
| | *Candida* sp., *Penicillium* sp. |
| | Aspergillus sp. |

**Table 2. Classification of biosurfactants**

| | |
|---|---|
| **1.** | **Glycolipids** |
| | Trehalose lipids |
| | Sophorolipids |
| | Rhamnolipids |
| **2.** | **Fatty Acids** |
| **3.** | **Phospholipids** |
| **4.** | **Surfactant active antibodies** |
| | Gramicidin S |
| | Polymixins |
| | Surfactin |
| | Antibiotic TA |
| **5.** | **Polymeric microbial surfactant** |
| | Emulsan from *Acinetobacter calcoaceticus* RAG-1 (ATCC 31012) |
| | The polysaccharide protein complex of *Acinetobacter calcoaceticus* BD4 |
| | *Other Acinetobacter sp.* emulsiliers |
| | Polysaccharide liquid complexes from yeasts |
| | Emulsifying protein PA from *Pseudomonas aeruginosa* |
| | Emulsifying and solubilizing factors from *Pseudomonas sp.* PG-1 |
| | Bioflocculant and emulcyan from the filamentous *Cyanobacterium phormidium* 3-1 |
| **6.** | **Particulate surfactants** |
| | Extracellular vesicles from *Acinetobacter* sp. H01-N |
| | Microbial cells with high cell surface hydrophobicities |

A particularly preferred biosurfactant produced by the method of the present invention is a rhamnolipid. As indicated above, in the context of the invention a "rhamnolipid" refers to a glycolipid that has a lipid portion that includes one or more, typically linear, saturated or unsaturated β-hydroxy-carboxylic acid moieties and a saccharide portion of one or more units of rhamnose or an ester thereof. The saccharide portion and the lipid portion are linked via an O-glycosidic bond between the 1-OH group of a rhamnose-moiety of the saccharide portion and the 3-OH group of a β-hydroxy-carboxylic acid of the lipid portion. Thus the carboxylic group of one carboxylic acid moiety defines the end of the rhamnolipid. This carboxylic group may be either a free carboxylic group or it may define an ester with an aliphatic alcohol. Where more than one rhamnose-moiety is included in a rhamnolipid, each of the rhamnose moieties not linked to the lipid portion is linked to another rhamnose moiety via an 1,2-glycosidic bond. Thus the 3-OH group of a rhamnose-moiety can be taken to define an end of the rhamnolipid. This hydroxy group may be either a free hydroxy group or it may define an ester with an aliphatic carboxylic acid. In embodiments where two or more β-hydroxy-carboxylic acids are present in a rhamnolipid, the β-hydroxy-carboxylic acid moieties are selected independently from each other. β-hydroxy-carboxylic acid moieties of a respective plurality of β-hydroxy-carboxylic acid moieties may be in some embodiments be identical. In some embodiments they are different from each other.

Generally a rhamnolipid can be represented by the following formula (I).

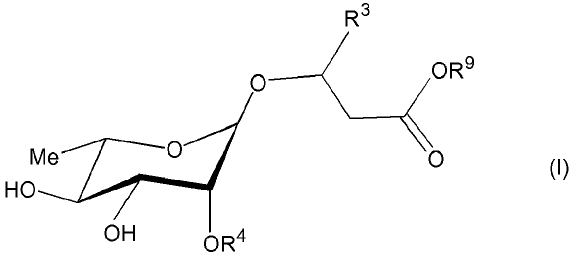

In this formula R⁹ is a hydrogen atom (H) or an aliphatic group that has a main chain of one to about 46, such as one to about 42, one to about 40, one to about 38, one to about 36, one to about 34, one to about 30, one to about 28, including e.g. 2, 3, 4, 5, 6, 7, 8, 9, 10, 11, 12, 13, 14, 15, 16, 17, 18, 19, 20, 21, 22, 23, 24, 25, 26, 27 or 28 carbon atoms and one to about three, including two, oxygen atoms. In some embodiments the main chain of the respective aliphatic group carries a terminal carboxylic acid group and/or an internal ester group. As an illustrative example in this regard, R⁹ may be of the formula-CH(R⁵)-CH₂-COOR⁶, including of the formula -CH(R⁵)-CH₂-COO-CH(R⁷)-CH₂-COOR⁸. In these illustrative moieties, R⁵ may be an aliphatic moiety with a main chain that has a length from 1 to about 19, such as from 1 to about 17, from 1 to about 15, from 1 to about 13, about 2 to about 13, about 3 to about 13 or about 4 to about 13, including e.g. 2, 3, 4, 5, 6, 7, 8, 9, 10, 11 or 12 carbon atoms. R⁶ and R⁸ are independent from one another a hydrogen atom (H) or an aliphatic group that has a main chain of one to about five, such as 2, 3 or 4 carbon atoms. R⁷ is a hydrogen atom (H) or an aliphatic group that has a main chain of one to about 19 carbon atoms, such as two to about 19 or three to about 19, e.g. 3, 4, 5, 6, 7, 8, 9, 10, 11, 12, 13, 14, 15, 16, 17 or 18 carbon atoms.

The term "aliphatic" means, unless otherwise stated, a straight or branched hydrocarbon chain, which may be saturated or mono- or poly-unsaturated and include heteroatoms. The term "heteroatom" as used herein means an atom of any element other than carbon or hydrogen. An unsaturated aliphatic group contains one or more double and/or triple bonds (alkenyl or alkinyl moieties). The branches of the hydrocarbon chain may include linear chains as well as non-aromatic cyclic elements. The hydrocarbon chain, which may, unless otherwise stated, be of any length, and contain any number of branches. Typically, the hydrocarbon (main) chain includes 1 to 5, to 10, to 15 or to 20 carbon atoms. Examples of alkenyl radicals are straight-chain or branched hydrocarbon radicals which contain one or more double bonds. Alkenyl radicals generally contain about two to about twenty carbon atoms and one or more, for instance two, double bonds, such as about two to about ten carbon atoms, and one double bond. Alkynyl radicals normally contain about two to about twenty carbon atoms and one or more, for example two, triple bonds, such as two to ten carbon atoms, and one triple bond. Examples of alkynyl radicals are straight-chain or branched hydrocarbon radicals which contain one or more triple bonds. Examples of alkyl groups are methyl, ethyl, propyl, butyl, pentyl, hexyl, heptyl, octyl, nonyl, decyl, the n isomers of these radicals, isopropyl, isobutyl, isopentyl, sec-butyl, tert-butyl, neopentyl, 3,3-dimethylbutyl. Both the main chain as well as the branches may furthermore contain heteroatoms as for instance N, O, S, Se or Si or a carbon atom may be replaced by one of these heteroatoms. An aliphatic moiety may be substituted or unsubstituted with one or more functional groups. Substituents may be any functional group, as for example, but not limited to, amino, amido, carbonyl, carboxyl, hydroxyl, nitro, thio and sulfonyl.

A R⁴ in formula (I) is a hydrogen atom (H), a substituted or unsubstituted rhamnopyranosyl moiety, or an aliphatic group having a main chain of one to about 12, such as 2, 3, 4, 5, 6, 7, 8, 9, 10 or 11 carbon atoms that may be saturated or unsaturated or an acyl group -C(O)R¹⁰, wherein R¹⁰ is a hydrogen atom (H) an aliphatic group having a main chain of one to about 11, such as 2, 3, 4, 5, 6, 7, 8, 9 or 10 carbon atoms. A respective aliphatic group of R² may include a keto group at the α-position. In the above formulae n is an integer selected in the range from 1 to about 17, such as from 1 to about 15, from 1 to about 13, about 2 to about 13, about 3 to about 13 or about 4 to about 13, including e.g. 2, 3, 4, 5, 6, 7, 8, 9, 10, 11 or 12. β-hydroxy-carboxylic acid moieties of a respective plurality of β-hydroxy-carboxylic acid moieties may be identical or different. Where R⁴ is a substituted rhamnopyranosyl moiety, it is typically substituted at the hydroxyl group at the 2 position in the form of an ester group or an ether group replacing the hydroxyl group. A respective ester group may include an aliphatic moiety with a main chain of one to about 11, such as 2, 3, 4, 5, 6, 7, 8, 9 or 10 carbon atoms. A corresponding ether group may be a further substituted or unsubstituted rhamnopyranosyl moiety or include an aliphatic group that has a main chain of one to about 12, such as 2, 3, 4, 5, 6, 7, 8, 9, 10 or 11 carbon atoms that may be saturated or unsaturated.

R³ in the above formula (I) is an aliphatic group having a main chain of about 3 to about 19, such as 4, 5, 6, 7, 8, 9, 10, 11, 12, 13, 14, 15, 16, 17 or 18 carbon atoms. In typical embodiments where a rhamnolipid includes only saturated β-hydroxy-carboxylic acid moieties a respective rhamnolipid can be represented by one of the following general formulae (II), (III), (IV), (V) or (VI):

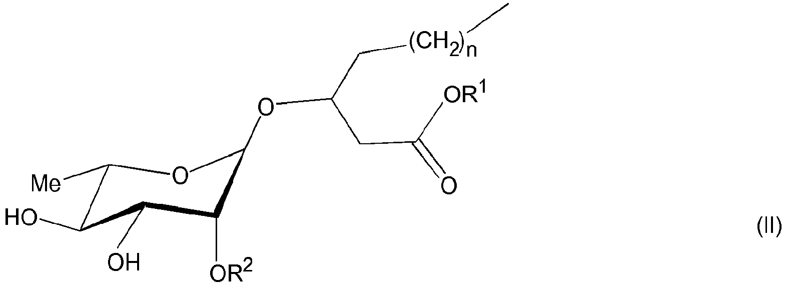

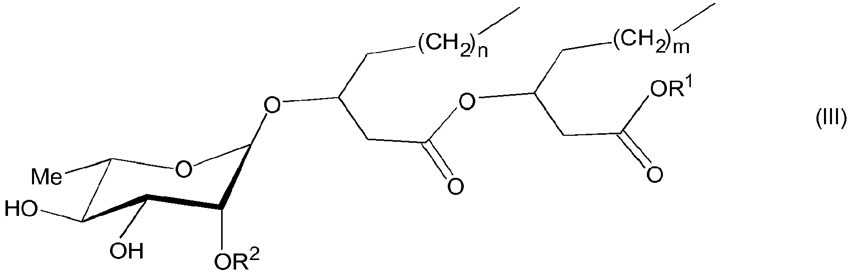

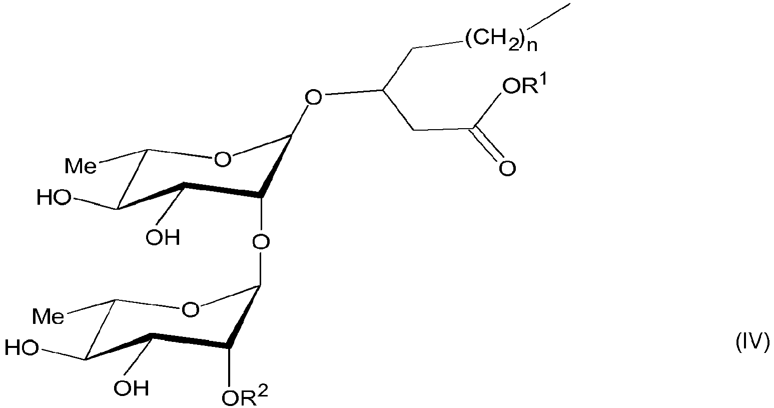

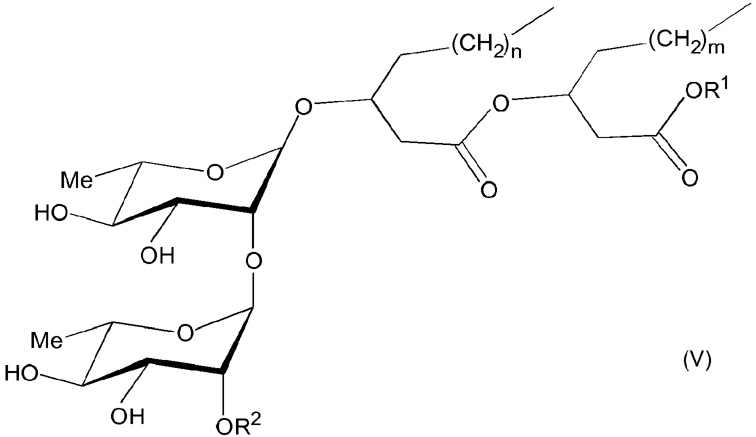

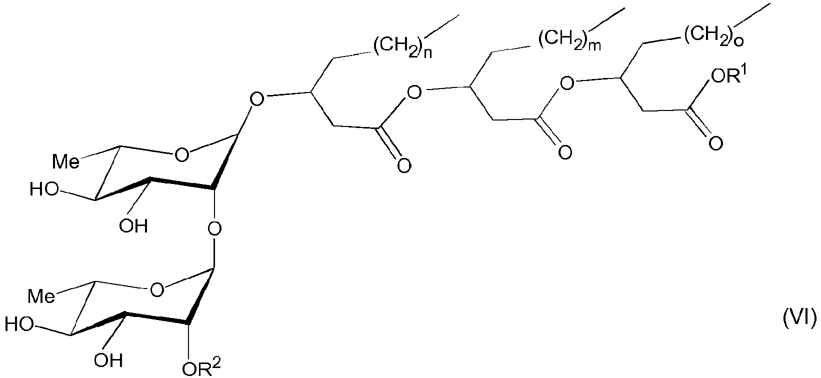

R¹ in the above formulae is a hydrogen atom (H) or an aliphatic group having a main chain of one to about five, such as 2, 3 or 4 carbon atoms. R² in the above formulae is a hydrogen atom (H), an aliphatic group having a main chain of one to about 12, such as 2, 3, 4, 5, 6, 7, 8, 9, 10 or 11 carbon atoms that may be saturated or unsaturated or an acyl group -C(O)R³, wherein R¹⁰ is a hydrogen atom (H) an aliphatic group having a main chain of one to about 11, such as 2, 3, 4, 5, 6, 7, 8, 9 or 10 carbon atoms. A respective aliphatic group of R² may include a keto group at the α-position. In the above formulae n is an integer selected in the range from 1 to about 17, such as from 1 to about 15, from 1 to about 13, about 2 to about 13, about 3 to about 13 or about 4 to about 13, including e.g. 2, 3, 4, 5, 6, 7, 8, 9, 10, 11 or 12. Likewise in formulae (III), (V), and (VI) m is an integer selected in the range from 1 to about 17, such as from 1 to about 15, from 1 to about 13, about 2 to about 13, about 3 to about 13 or about 4 to about 13, including e.g. 2, 3, 4, 5, 6, 7, 8, 9, 10, 11 or 12. In formula (VI) o is an integer selected in the range from 1 to about 17, such as from 1 to about 15, from 1 to about 13, about 2 to about 13, about 3 to about 13 or about 4 to about 13, including e.g. 2, 3, 4, 5, 6, 7, 8, 9, 10, 11 or 12.

In embodiments where a rhamnolipid includes one or more unsaturated β-hydroxy-carboxylic acid moieties a respective rhamnolipid may resemble the above formulas, however, include one or more unsaturated carboxylic acid moieties instead of the saturated carboxylic acid moieties depicted above. Such a rhamnolipid may for instance include a 3-hydroxy-n-octenoic acid moiety, a 3-hydroxy-n-octadienoic acid moiety, a 3-hydroxy-n-decenoic acid moiety, a 3-hydroxy-n-dodecenoic acid moiety, a 3-hydroxy-n-dodecadienoic acid moiety, a 3-hydroxy-n-tetradecenoic acid moiety or a 3-hydroxy-n-tetradecadienoic acid moiety (e.g. Arutchelvi, J., & Doble, M., J. Letters in Applied Microbiology (2010) 51, 75-82; Sharma, A., et al., J. Nat. Prod. (2007) 70, 941-947).

The method of the present invention is preferably carried out in a reactor such as a bioreactor, sometimes herein also called fermentor. A bioreactor refers to any manufactured or engineered device, container, receptacle, or system that supports a biologically active environment. A manufacturing (production) process in a bioreactor can either be aerobic or anaerobic. These bioreactors are, for example, cylindrical, ranging in size from liters to cubic meters, and are often made of stainless steel. On the basis of mode of operation, a bioreactor may be classified as batch, fed batch or continuous (e.g. a continuous stirred-tank reactor model). An apparatus used to carry out any kind of bioprocess; examples include fermentor or enzyme reactor.

The method of the present invention is preferably scalable. Scalable includes lab-scale, pilot-scale and industrial scale. As used herein, "lab-scale" includes performing the methods of the present invention (including culturing and allowing the formation and/or accumulation of foam) in a reactor which is capable of encompassing 1-10 liters such as 1, 2, 3, 4, 5, etc. liters culture broth. As used herein, "pilot-scale" includes performing the methods in a reactor capable of comprising 10-100 liters such as 10, 20, 30, 40, 50, or 100 liters culture broth. As used herein, "industrial scale" or large-scale includes performing the methods in a reactor capable of encompassing 100-3000 liters such as 200, 300, 400, 500, 1000, 2000, 3000 liters culture broth.

For the culture process in the present invention, any medium can generally be used, provided that it contains assimilable nutritional sources for cells producing biosurfactants. For example, usable are common media appropriately containing materials mixed therein. Examples of the materials include carbon sources such as sugars (e.g. glucose, sucrose, maltose), organic acids (e.g. lactic acid, acetic acid, citric acid, propionic acid), alcohols (e.g. ethanol, glycerin), and mixtures of these; nitrogen sources such as ammonium sulfate, ammonium phosphate, urea, yeast extract, meat extract, peptone, and corn steep liquor; and other nutritional sources such as mineral salts and vitamins. Other media that are appropriate, in particular for the production of rhamnolipids are described in Heyd (2009), Dissertation "Continuous production of rhamnolipids by means of process integration". Media suitable for the production of biosurfactants are described in Desai and Banat (1997), Microbiol Molec Biol Rev 61(1):47-64.

A sugar and/or other hydrophilic compound, mixture of compounds or substance may be used as a main raw material. Glucose is the preferred sugar as a main raw material. Examples of hydrophilic substances as a main raw material include compounds which easily can be metabolized like citric acid, succinic acid, glycerol etc..

Further parameters for culturing cells producing biosurfactants such as speed of the stirrer, air flow rate, initial air bubble size, foam height, operation time, etc. are described in US 4,628,030; US 4,933,281; Chen et al. (2006); Sarachat et al. (2010); Heyd (2009); Heyd et al. (2011), all cited herein.

A "host cell" or, sometimes also referred as "cell" applied in the method of the present invention includes any suitable (host) cell that is capable of producing a biosurfactant, i.e. a biosurfactant producing cell. A cell producing a biosurfactant can be a naturally occurring cell that is preferably an isolated cell when applied in the method of the present invention or a recombinant cell. Encompassed are thus those cells which are capable of producing a biosurfactant as mentioned herein, for example, a biosurfactant as listed in Table 1. A cell as applied in the method of the present invention also encompasses recombinant cells or genetically engineered cells. For example, if the gene(s) encoding the protein(s) required for the synthesis of a biosurfactant is/are known, that gene(s) can be cloned into an appropriate host cells, i.e., into a heterologous host cell. Heterologous host cells means that the host cell is different from the cell from which the gene(s) encoding the protein(s) required for the synthesis of a biosurfactant originates. A particularly preferred cell is one which is capable of producing a rhamnolipid.

Further preferred host cells encompassed by the present invention producing preferred biosurfactants encompassed by the present invention are listed in Table 3 below:

**Table 3**

| Biosurfactant | Organisms |
|---|---|
| Glycolipids | |
| Rhamnolipids | *P. aeruginosa* |
| | *Pseudomonas* sp. |
| Trehalolipids | *R. erythropolis* |
| | *N*. *erythropolis* |
| | *Mycobacterium* sp. |
| Sophorolipids | *T*. *bombicola* |
| | *T*. *apicola* |
| | *T. petrophilum* |
| Cellobiolipids | *U. zeae, U. maydis* |
| | |
| Lipopeptides and lipoproteins | |
| Peptide-lipid | *B. licheniformis* |
| Serrawettin | *S*. *marcescens* |
| Viscosin | *P. fluorescens* |
| Surfactin | *B*. *subtilis* |
| Subtilisin | *B. subtilis* |
| Gramicidins | *B*. *brevis* |
| Polymyxins | *B. polymyxa* |
| | |
| Fatty acids, neutral lipids, and phospholipids | |
| Fatty acids | *C. lepus* |
| Neutral lipids | *N*. *erythropolis* |
| Phospholipids | *T*. *thiooxidans* |
| | |
| Polymeric surfactants | |
| Emulsan | *A. calcoaceticus* |
| Biodispersan | *A*. *calcoaceticus* |
| Mannan-lipid-protein | *C. tropicalis* |
| Liposan | *C. lipolytica* |
| Carbohydrate-protein-lipid | *P*. *fluorescens* |
| | *D*. *polymorphis* |
| Protein PA | *P. aeruginosa* |
| | |
| Particulate biosurfactants | |
| Vesicles and fimbriae | *A*. *calcoaceticus* |
| Whole cells | Variety of bacteria |

The present invention envisages as a biosurfactant producing cell preferably non-pathogenic host cells (non-pathogenic for humans) including a unicellular host cell such as a fungal host cell, for example, a yeast. Preferably, however, the cell of the present invention is a prokaryotic cell. Prokaryotic cells encompass bacterial host cells including non-pathogenic bacterial cells such as bacterial host cells capable of producing a biosurfactant. In a preferred embodiment, the host cell is a gram-negative cell.

A particularly preferred host cell is one elected from the group consisting of *Pseudomonas putida, Pseudomonas chlororaphis, Pseudomonas fluorescens, Pseudomonas alcaligenes, Pseudomonas aeruginosa, Pseudomonas cepacia, Pseudomonas clemancea, Pseudomonas collierea, Pseudomonas luteola, Pseudomonas stutzeri, Pseudomonas teessidea, Escherichia coli, Renibacterium salmoninarum, Cellulomonas cellulans, Tetragenococcus koreensis, Burkholderia glumae, Burkholderia mallei, Burkholderia pseudomallei, Burkholderia plantarii, Burkholderia thailandensis, Acinetobacter calcoaceticus, Enterobacter asburiae, Enterobacter hormaechei, Pantoea* stewartii and *Pantoea ananatis.*

A preferred cell applied in the method of the present invention includes (i) a *rhlA* gene or an ortholog thereof; and (ii) a *rhlB* gene or an ortholog thereof.

Any rhlA gene may be included in a cell according to the invention. Examples include, but are not limited to, a gene encoding the rhlA protein of Pantoea ananatis, strain LMG 20103, with SwissProt accession no. D4GK92 (Version 6 of 11 January 2011), of Pantoea ananatis AJ13355, with SwissProt accession no. F2EY06 (Version 1 of 31 May 2011), Pseudomonas aeruginosa, with SwissProt accession no. Q51559 (30 November 2010, version 60), of Burkholderia thailandensis, strain E264 / ATCC 700388 / DSM 13276 / CIP 106301, SwissProt accession no. Q2T424 (version 25 of 30 November 2010), of Burkholderia pseudomallei, strain 1106a, SwissProt accession no. A3P349 (version 19 of 11 January 2011), of Burkholderia pseudomallei, strain 1710a, SwissProt accession no. C6U4Y4 (version 5 of 11 January 2011), of Burkholderia pseudomallei, strain 1710b, SwissProt accession no. Q3JGQ8 (version 30 of 11 January 2011), of Burkholderia pseudomallei 1106b, SwissProt accession no. C5ZMA0 (Version 4 of 11 January 2011), of Burkholderia pseudomallei, strain 668, SwissProt accession no. A3NHI8 (version 20 of 11 January 2011), Burkholderia pseudomallei 406e, SwissProt accession no. A8EAW6 (version 5 of 11 January 2011), of Burkholderia mallei, SwissProt accession no. Q62CH3 (Version 32 of 11 January 2011), of Burkholderia mallei, strain SAVP1, SwissProt accession no. A1UVS0 (version 18 of 11 January 2011), of Burkholderia mallei, strain NCTC 10247, SwissProt accession no. A3MEC2, (version 19 of 11 January 2011), of Burkholderia mallei JHU, SwissProt accession no. A5XJN3 (Version 7 of 11 January 2011), of Burkholderia glumae, strain BGR1, SwissProt accession no. C5AMF7 (version 9 of 30 November 30), of Burkholderia gladioli BSR3, SwissProt accession no. F2LKI9 (version 1 of 31 May 2011), of Burkholderia ambifaria, strain MC40-6, SwissProt accession no. B1Z031 (version 13 of 30 November 2010) of Dickeya dadantii, strain 3937, SwissProt accession no. E0SMT5 (version 5of 5 April 2011), of Pseudomonas fluorescens, strain SBW25, SwissProt accession no. C3K3D6 (version 10 of 11 January 2011), of Pseudomonas sp. DHT2, SwissProt accession no. A1YW88 (Version 5 of 19 January 2010) and of Pseudomonas aeruginosa, strain PA7, SwissProt accession no. A6V1 U6 (version 19 of 30 November 2010), to name a few. As four examples of a respective rhlA gene may serve the gene of EMBL-Bank accession no. CP000744.1 of Pseudomonas aeruginosa PA7, the gene of NCBI Gene ID 4888867 of Burkholderia pseudomallei strain 668, the gene of NCBI GeneID 8894591 of the Pantoea ananatis LMG 20103 chromosome (NCBI reference sequence NC_013956.2), the gene of NCBI GeneID:9733431 of the Dickeya dadantii 3937 chromosome (NCBI reference sequence NC_014500.1).

Further proteins have been identified that are likely to define rhamnosyltransferase 1A subunits. A gene encoding such a protein can likewise be employed as long as it results in the formation of a functional rhamnosyltransferase subunit. Based on sequence similarity on the protein level, examples of genes encoding probable rhamnosyltransferase 1A subunits include, but are not limited to, a gene encoding the protein of Pseudomonas putida, strain W619, SwissProt accession no. B1J4I8 (version 14 of 30 November 2010), the protein of Pseudomonas mendocina, strain ymp, SwissProt accession no. A4XS03 (version 20 of 31 May 2011), the protein of Pseudomonas sp. TJI-51, SwissProt accession no. F0E3C8 (version 2 of 31 May 2011), the protein of Pseudomonas sp. DHT2, SwissProt accession no. A1YW88 (version 5 of 19 January 2010), the protein of Pseudomonas syringae pv. Phaseolicola, strain 1448A / Race 6, SwissProt accession no. Q48HB4 (Version 29 of 11 January 2011), the protein of Pseudomonas savastanoi pv. savastanoi NCPPB 3335, SwissProt accession no. D7I4I4 (version 2 of 5 April 2011), the protein of Pseudomonas sp. USM 4-55, SwissProt accession no. B7SJG2 (version 4 of 10 August 2010), the protein of Pseudomonas nitroreducens, SwissProt accession no. Q93LI7 (version 18 of 5 October 2010), the protein of Pseudomonas entomophila, strain L48, SwissProt accession no. Q1I5S9 (version 27 of 11 January 2011), the protein of Pseudomonas brassicacearum subsp. brassicacearum NFM421, SwissProt accession no. F2KE24 (version 1 of 31 May 2011), the protein of Pseudomonas stutzeri (Pseudomonas perfectomarina), SwissProt accession no. Q8KSD5 (version 1 of 5 October 2010), the protein of Pseudomonas fluorescens, SwissProt accession no. B1 PWE2 (version 6 of 5 October 2010), the protein of Pseudomonas oleovorans, SwissProt accession no. Q9KJH8 (version 33 of 31 May 2011), the protein of Pseudomonas sp. USM 4-55, SwissProt accession no. B7SJG2 (version 4 of 10 August 2010), the protein of Pseudomonas pseudoalcaligenes, SwissProt accession no. Q93MS5 (version 25 of 5 October 2010), the protein of Burkholderia ambifaria, strain MC40-6, SwissProt accession no. B1Z031 (version 1 of 30 November 2010), the protein of Burkholderia ambifaria, strain ATCC BAA-244 / AMMD, SwissProt accession no. Q0B714 (version 22 of 11 January 2011), the protein of Burkholderia ambifaria MEX-5, SwissProt accession no. B1T5A9 (version 5 of 10 August 2010), the protein of Burkholderia ambifaria IOP40-10 with SwissProt accession no. B1FHM8 (version 6 of 5 October 2010), the protein of Burkholderia sp. TJI49, SwissProt accession no. F0GF54 (Version 2 of 31 May 2011), the protein of Burkholderia cenocepacia, strain AU 1054, SwissProt accession no. Q1 BQD9 (Version 21 of 30 November 2010), the protein of Burkholderia cenocepacia, strain MC0-3, SwissProt accession no. B1K710 (30 November 2010), the protein of Burkholderia cepacia, strain J2315 / LMG 16656, SwissProt accession no. B4EHI9 (version 13 of 11 January 2011), the protein of Burkholderia sp. strain 383 (Burkholderia cepacia strain ATCC 17760 / NCIB 9086 / R18194 (version 26 of 30 November), the protein of Burkholderia caryophylli, SwissProt accession no. Q93LI6 (October 5, 2010. Version 20), the protein of Burkholderia ubonensis Bu, NCBI accession no. ZP_02376540.1 (as of 9 December 2010), the protein of Brevundimonas sp. BAL3, SwissProt accession no. B4WER6 (version 6 of 10 August 2010), the protein of Acidovorax ebreus, strain TPSY, SwissProt accession no. B9MA04 (version 12 of 30 November 2010), the protein of Acidovorax sp. strain JS42, SwissProt accession no. A1W249 (version 26 of 30 November 2010), the protein of Dickeya dadantii, strain Ech703, SwissProt accession no. C6C8B4 (version 8 of 30 November 2010), the protein of Dickeya dadantii, strain Ech586, SwissProt accession no. D2C1 P1 (version 7 of 30 November 2010), the protein of Dickeya dadantii, strain 3937 (Erwinia chrysanthemi, strain 3937), SwissProt accession no. E0SMT5 (Version 5 of 5 April 2011), the protein of Dickeya zeae, strain Ech1591, SwissProt accession no. C6CKC2 (version 8 of 30 November 2010), the protein of Serratia odorifera DSM 4582, SwissProt accession no. D4E5A8 (version 4 of 5 April 2011), the protein of Nocardia farcinica with SwissProt accession no. Q5YPG5 (version 35 of 30 November 2010), the protein of Anaeromyxobacter dehalogenans, strain 2CP-C, with SwissProt accession no. Q2IK44 (version 33 of 30 November 2010), the protein of Anaeromyxobacter dehalogenans, strain 2CP-1 / ATCC BAA-258, with SwissProt accession no. B8J5U1 (version 11 of 30 November 2010), the protein of Amycolatopsis mediterranei, strain U-32, with SwissProt accession no. D8I794 (version 4 of 11 January 2011) and the protein of Halothiobacillus neapolitanus, strain ATCC 23641 / c2 (Thiobacillus neapolitanus), SwissProt accession no. D0KWX9 (version 6 of 30 November 2010).

Any rhlB gene may be included in a cell according to the invention. Examples include, but are not limited to, a gene encoding the rhlB protein of *Pseudomonas aeruginosa,* with SwissProt accession no. D2EDM4 (version 5 of 8 March 2011), of *Pseudomonas aeruginosa,* strain UCBPP-PA14, with SwissProt accession no. Q02QW7 (version 27 of 8 March 2011), of *Pseudomonas aeruginosa,* strain PA7, with SwissProt accession no. A6V1 U7 (Version 23 of 8 March 2011), of *Pseudomonas sp. BSFD5,* with SwissProt accession no. D9IV58 (Version 4 of 8 March 2011), of *Pseudomonas aeruginosa* 2192 with SwissProt accession no. A3LDS3 (Version 17 of 8 March 2011), of *Burkholderia mallei,* strain SAVP1, with SwissProt accession no. A1UVR8 (version 20 of 8 March 2011), of *Burkholderia mallei* ATCC 10399, SwissProt accession no. A9K2T0 (version 14 of 8 March 2011), of *Burkholderia mallei JHU,* SwissProt accession no. A5XJN5 (version 14 of 8 March 2011), of *Burkholderia mallei PRL-20,* SwissProt accession no. C5NA24 (version 5 of 8 March 2011), of *Burkholderia pseudomallei,* strain 1106a, SwissProt accession no. A3P351 (Version 21 of 8 March 2011), of *Burkholderia pseudomallei,* strain 1106b, SwissProt accession no. C5ZMA2 (Version 6 of 8 March 2011), of *Burkholderia thailandensis,* strain E264 / ATCC 700388 / DSM 13276 / CIP 106301, SwissProt accession no. Q2T425 (Version 32 of 8 March 2011), of *Dickeya dadantii,* strain 3937 (Erwinia chrysanthemi, strain 3937), SwissProt accession no. E0SJM9 (Version 6 of 5 April 2011), of *Pantoea ananatis* AJ13355, SwissProt accession no. F2EY05 (Version 1 of 13 May 2011), of *Pantoea ananatis,* strain LMG 20103, SwissProt accession no. D4GK91 (Version 7 of 8 March 2011), of *Blastopirellula marina DSM* 3645, SwissProt accession no. A4A1V5 (Version 13 of 8 March 2011) and of *Acidobacterium sp. MP5ACTX8,* SwissProt accession no. D6UX52 (Version 3 of 11 January 2011).

As a few examples of a respective rhlB gene may serve the *Pantoea ananatis LMG* 20103gene of EMCBI Gene ID 8894590 (as of 12 May 2011), the *Pseudomonas aeruginosa* PAO1 gene of EMCBI Gene ID 878954 (as of 10 March 2011), the *Burkholderia pseudomallei* 1106a gene of EMCBI Gene ID 4905917 (as of 14 January 2011), the *Burkholderia mallei,* strain SAVP1, gene of EMCBI Gene ID 4678088 (as of 12 March 2010), the *Burkholderia mallei,* strain ATCC 23344, gene of EMCBI Gene ID 3086474 (as of 22 March 2011), the *Burkholderia mallei,* strain ATCC 23344, gene of EMCBI Gene ID 3087541 (as of 22 March 2011).

Similar to the rhamnosyltransferase 1A protein, further proteins have been identified that are likely to define rhamnosyltransferase 1B subunits. A gene that encodes such a protein can likewise be employed as long as it results in the formation of a functional rhamnosyltransferase subunit. On the basis of sequence similarity on the protein level, examples of genes encoding probable rhamnosyltransferase 1B subunits include, but are not limited to, a gene encoding the protein of *Burkholderia pseudomallei* with SwissProt accession no. Q63KL0 (Version 35 of 8 March 2011), the protein of *Burkholderia pseudomallei 305,* SwissProt accession no. A4LRW4 (Version 13 of 11 January 2011), the protein of *Burkholderia cenocepacia,* strain HI2424, SwissProt accession no. A0B2F2 (Version 24 of 8 March 2011), the protein of *Burkholderia cenocepacia,* strain MC0-3, SwissProt accession no. B1K712 (Version 13 of 8 March 2011), the protein of *Burkholderia cepacia,* strain J2315 / LMG 16656 (Burkholderia cenocepacia, strain J2315), SwissProt accession no. B4EHI7 (Version 13 of 8 March 2011), the protein of *Burkholderia cenocepacia,* strain AU 1054, SwissProt accession no. Q1 BQD7 (Version 31 of 8 March 2011), the protein of *Burkholderia ambifaria,* strain ATCC BAA-244 / AMMD, (*Burkholderia cepacia,* strain AMMD), SwissProt accession no. Q0B716 (Version 28 of 8 March 2011), the protein of *Burkholderia glumae,* strain BGR1, SwissProt accession no. C5AMF8 (Version 10 of 8 March 2011), the protein of *Burkholderia gladioli BSR3,* SwissProt accession no. F2LT33 (Version 1 of 31 May 2011), the protein of *Burkholderia sp. TJI49,* SwissProt accession no. F0GF56 (Version 2 of 31 May 2011), the protein of *Burkholderia multivorans* CGD2M with SwissProt accession no. B9C4N0 (Version 6 of 8 May 2011), the protein of *Dickeya dadantii,* strain Ech586, SwissProt accession no. D2BRY4 (Version 8 of 8 March 2011), the protein of *Dickeya dadantii,* strain Ech703, SwissProt accession no. C6C959 (Version 9 of 8 March 2011), the protein of *Dickeya zeae,* strain Ech1591, SwissProt accession no. C6CEW6 (Version 9 of 8 March 2011), the protein of *Polaromonas sp.* strain JS666 / ATCC BAA-500, SwissProt accession no. Q121J6 (Version 32 of 8 March 2011), the protein of *Methylobacterium extorquens,* strain PA1, SwissProt accession no. A9W4M1 (Version 19 of 8 March 2011), the protein of *Methylocystis sp.* ATCC 49242, SwissProt accession no. E8KZV1 (Version 2 of 31 May 2011), the protein of Methylobacterium *chloromethanicum,* strain CM4 / NCIMB 13688, SwissProt accession no. B7L372 (Version 12 of 8 March 2011), the protein of *Acidobacterium sp.* MP5ACTX8, SwissProt accession no. D6UZE1 (Version 4 of 8 March 2011), the protein of *Acidobacterium capsulatum,* strain ATCC 51196 / DSM 11244 / JCM 7670, SwissProt accession no. C1F8F6 (Version 11 of 8 March 2011), the protein of *Solibacter usitatus,* strain Ellin6076, SwissProt accession no. Q023U1 (Version 25 of 8 March 2011) and the protein of *Maritimibacter alkaliphilus* HTCC2654, SwissProt accession no. A3VBK0 (Version 15 of 8 March 2011).

Preferably, the *rhlA* gene or ortholog thereof and/or the *rhlB* gene or ortholog thereof are under the control of a heterologous promoter.

In another preferred embodiment, the cell further includes a *rhlC* gene or an ortholog thereof. Preferably, the *rhlC* gene or ortholog thereof is under the control of a heterologous promoter.

A *rhlC* gene included in the bacterial host cell according to the invention may be any *rhlC* gene. Examples of a suitable gene include, but are not limited to, a gene encoding the rhlC protein of *Pseudomonas aeruginosa* with SwissProt accession no. D2EDP8 (version 6 of 31 May 2011), of *Pseudomonas aeruginosa,* strain UCBPP-PA14 with SwissProt accession no. Q02IV0 (Version 22 of 31 May 2011), of Pseudomonas aeruginosa with SwissProt accession no. D2EDQ3 (Version 4 of 31 May 2011), of Burkholderia mallei ATCC 10399 with SwissProt accession no. A9K2T2 (Version 14 of 31 May 2011), of Burkholderia mallei, strain NCTC 10247, SwissProt accession no. A3MEB8 (Version 21 of 31 May 2011), of *Burkholderia pseudomallei Pasteur* 52237 with SwissProt accession no. A8KHX2 (Version 13 of 31 May 2011) and of *Burkholderia pseudomallei* S13 with SwissProt accession no. B1 HLL2 (Version 8 of 31 May 2011).

Further rhamnosyltransferases have been identified that are, based on sequence identity, likely a rhamnosyltransferase-2 and thus encoded by a *rhlC* gene. A gene that encodes a respective protein may likewise be used as a *rhlC* gene as long as it results in the formation of a functional rhamnosyltransferase. Examples of a gene encoding a probable rhamnosyltransferase-2 subunits include, but are not limited to, a gene encoding the protein of Burkholderia thailandensis, strain E264 / ATCC 700388 / DSM 13276 / CIP 106301 with SwissProt accession no. Q2T428 (version 26 of 31 May 2011), the protein of Burkholderia pseudomallei with SwissProt accession no. Q63MV9 (version 26 of 31 May 2011), of Burkholderia glumae, strain BGR1 with SwissProt accession no. C5AMG0 (version 9 of 31 May 2011), of Burkholderia glumae, strain BGR1, with SwissProt accession no. C5ABW1 (version 8 of 30 November 2010), of Burkholderia gladioli BSR3 with SwissProt accession no. F2LKJ2 (version 1 of 31 May 2011), of Burkholderia cenocepacia, strain MC0-3, SwissProt accession no. B1 K714 (version 14 of 31 May 2011), of Burkholderia cenocepacia PC184, SwissProt accession no. A2W519 (version 14 of 5 October 2010), of Burkholderia ambifaria, strain MC40-6, SwissProt accession no. B1Z027 (Version 14 of 31 May 2011), of Burkholderia sp. TJI49 with SwissProt accession no. F0G014 (version 2 of 31 May 2011), of Burkholderia phytofirmans, strain DSM 17436 / PsJN, SwissProt accession no. B2T0J7 (version 13 of 31 May 2011), of Burkholderia phymatum, strain DSM 17167 / STM815, SwissProt accession no. B2JFC2 (version 12 of 30 November 2010), of Burkholderia multivorans CGD2M, SwissProt accession no. B9CFN7 (version 3 of 1 September 2009), of Lautropia mirabilis ATCC 51599, SwissProt accession no. E7RXL2 (version 2 of 31 May 2011), of Variovorax paradoxus EPS, SwissProt accession no. E6UV89 (version 2 of 5 April 2011), of Ralstonia solanacearum (Pseudomonas solanacearum), SwissProt accession no. Q8Y1 K3 (version 37 of 30 November 2010), of Ralstonia sp. 5_7_47FAA, SwissProt accession no. E2SY52 (version 3 of 31 May 2011), of Acidobacterium sp. MP5ACTX8, SwissProt accession no. D6UX48 (version 2 of 5 October 2010), of Klebsiella pneumonia, SwissProt accession no. C9K1 E5 (Version 3 of 20 April 2010), of Planctomyces maris DSM 8797, SwissProt accession no. A6C912 (version 10 of 31 May 2011), of Ralstonia pickettii, strain 12J, SwissProt accession no. B2U7B8 (version 14 of 31 May 2011), of Alteromonas macleodii, strain DSM 17117 / Deep ecotype, SwissProt accession no. F2GBW7 (version 1 of 31 May 2011), of Methylobacterium populi, strain ATCC BAA-705 / NCIMB 13946 / BJ001, SwissProt accession no. B1ZKT2 (version 15 of 30 November 2010), of Methylobacterium nodulans, strain ORS2060 / LMG 21967, SwissProt accession no. B8ISX9 (version 11 of 30 November 2010), of Methylobacterium chloromethanicum, strain CM4 / NCIMB 13688, SwissProt accession no. B7KW88 (version 11 of 30 November 2010), of Methylobacterium extorquens, strain PA1, SwissProt accession no. A9W727 (version 13 of 30 November 2010), of Methylobacterium radiotolerans, strain ATCC 27329 / DSM 1819 / JCM 2831, SwissProt accession no. B1M5I2 (version 14 of 30 November 2010), of Methylobacterium sp. strain 4-46, SwissProt accession no. B0ULR4 (version 12 of 30 November 2010), of Methylotenera mobilis, strain JLW8 / ATCC BAA-1282 / DSM 17540, SwissProt accession no. C6WVJ5 (version 9 of 31 May 2011), of Lautropia mirabilis ATCC 51599, SwissProt accession no. E7RXL2 (version 2 of 31 May 2011), of Acidovorax sp., strain JS42, SwissProt accession no. A1W3G6 (version 27 of 31 May 2011) and of Planctomyces maris DSM 8797, SwissProt accession no. A6C912 (version 10 of 31 May 2011).

Rhamnosyltransferase-2 catalyzes the transfer of a further rhamnosyl moiety to a mono-rhamnolipid, thereby providing a di-rhamnolipid. Accordingly, the presence of a *rhlC* gene and its control by a heterologous promoter may be desired in embodiments where the production of di-rhamnolipids is desired.

A host cell according to the invention includes a *rhlA* gene or an ortholog thereof. The *rhlA* gene or the respective ortholog is under the control of a heterologous promoter. In some embodiments the *rhlA* gene is an endogenous gene of the bacterial host cell. In some embodiments the *rhlA* gene is a heterologous gene. In some embodiments the *rhlA* gene or the respective ortholog is under the control of a promoter that is different from the promoter that controls the *rhlB* gene. In some embodiments the *rhlA* gene or the respective ortholog is under the control of a promoter that is similar or identical to the promoter that controls the *rhlB* gene. Likewise, the *rhlB* gene or the respective ortholog is under the control of a heterologous promoter. In some embodiments the *rhlB* gene is an endogenous gene of the bacterial host cell. In some embodiments the *rhlB* gene is a heterologous gene. Where present, the *rhlC* gene or the respective ortholog may in some embodiments be under the control of a heterologous promoter. In some embodiments the *rhlC* gene is an endogenous gene of the bacterial host cell. In some embodiments the *rhlC* gene is a heterologous gene. As should be apparent from the above, each of the promoters that controls the *rhlB* gene, the *rhlB* gene and in some embodiments the promoters that control the *rhlC* gene are selected independently from both the gene the respective promoter controls and from any other heterologous promoter that controls a gene of a rhamnosyltransferase peptide or protein.

The terms *"rhlA* gene" and *"rhlB* gene", as well as the term *"rhlC* gene", include variants. The term "variant" or "altered" in reference to a nucleic acid or polypeptide refers to polymorphisms, i.e. the exchange, deletion, or insertion of one or more nucleotides or amino acids, respectively, compared to the predominant form of the respective nucleic acid or polypeptide. A variant may be a polypeptide that includes a germline alteration. Such an alteration may be a deletion, insertion or substitution of one or more amino acids, and may include single nucleotide polymorphisms (SNPs). In the context of the present invention, a variant in some embodiments refers to a contiguous sequence of at least about 50, such as about 100, about 200, or about 300 amino acids set forth in the amino acid sequence of a protein named herein (cf. e.g. below), or the corresponding full-length amino acid sequence, with the proviso that the alteration is included in the respective amino acid sequence. In case the mutation leads to a premature stop codon in the nucleotide sequence encoding the protein, the sequence may even be shorter than the corresponding wild type protein. As a rough guidance, subunit A of rhamnosyltransferase I typically has an amino acid sequence with a length of about 200 to about 400, such as about 250 to about 350 amino acids, subunit B of rhamnosyltransferase I typically has an amino acid sequence with a length of about 350 to about 550 such as about 400 to about 500 amino acids, while rhamnosyltransferase II typically has an amino acid sequence with a length of about 100 to about 400, such as about 150 to about 350 amino acids. The rhamnosyltransferase polypeptide can be encoded by a full-length nucleic acid sequence, i.e. the complete coding sequence of the respective gene, or any portion of the full-length nucleic acid sequence, as long as the alteration of the polypeptide is retained.

The amino acid sequence of a variant is substantially similar to a known rhamnosyltransferase sequence such as a sequence referred to below. A sequence that is substantially similar to rhamnosyltransferase will in some embodiments have at least about 65 %, at least about 65 %, the amino acid sequence of a variant is substantially similar to a sequence referred to below. A sequence that is substantially similar to rhamnosyltransferase will in some embodiments have at least 60%, at least 70%, at least 80%, such as at least 90% identity, including at least 95%, at least 97%, at least 98%, at least 99%, or at least 99.5% identity to the sequence of a known rhamnosyltransferase, with the proviso that the altered position or sequence is retained.

By "identity" is meant a property of sequences that measures their similarity or relationship. Identity is measured by dividing the number of identical residues by the total number of residues and gaps and multiplying the product by 100. Preferably, identity is determined over the entire length of the sequences being compared. "Gaps" are spaces in an alignment that are the result of additions or deletions of amino acids. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity using standard parameters, for example Blast (Altschul, et al. (1997) Nucleic Acids Res. 25:3389-3402), Blast2 (Altschul, et al. (1990) J. Mol. Biol. 215:403-410), and Smith-Waterman (Smith, et al. (1981) J. Mol. Biol. 147:195-197). The term "mutated" or "mutant" in reference to a nucleic acid or a polypeptide refers to the exchange, deletion, or insertion of one or more nucleotides or amino acids, respectively, compared to the naturally occurring nucleic acid or polypeptide. The term "altered" or "variant" in reference to a nucleic acid or polypeptide refers to polymorphisms, i.e. the exchange, deletion, or insertion of one or more nucleotides or amino acids, respectively, compared to the predominant form of the respective nucleic acid or polypeptide.

The terms "polypeptide" and "protein" refer to a polymer of amino acid residues and are not limited to a certain minimum length of the product. Where both terms are used concurrently, this twofold naming accounts for the use of both terms side by side in the art.

The terms "nucleic acid" and "nucleic acid molecule" as used herein refer to any nucleic acid in any possible configuration, such as single stranded, double stranded or a combination thereof. Examples of nucleic acids include for instance DNA molecules, RNA molecules, analogues of the DNA or RNA generated using nucleotide analogues or using nucleic acid chemistry, locked nucleic acid molecules (LNA), protein nucleic acids molecules (PNA), alkylphosphonate and alkylphosphotri-ester nucleic acid molecules and tecto-RNA molecules (e.g. Liu, B., et al., J. Am. Chem. Soc. (2004) 126, 4076-4077). LNA has a modified RNA backbone with a methylene bridge between C4' and O2', providing the respective molecule with a higher duplex stability and nuclease resistance. Alkylphosphornate and alkylphosphotriester nucleic acid molecules can be viewed as a DNA or an RNA molecule, in which phosphate groups of the nucleic acid backbone are neutralized by exchanging the P-OH groups of the phosphate groups in the nucleic acid backbone to an alkyl and to an alkoxy group, respectively. DNA or RNA may be of genomic or synthetic origin and may be single or double stranded. Such nucleic acid can be e.g. mRNA, cRNA, synthetic RNA, genomic DNA, cDNA synthetic DNA, a copolymer of DNA and RNA, oligonucleotides, etc. A respective nucleic acid may furthermore contain non-natural nucleotide analogues and/or be linked to an affinity tag or a label.

Many nucleotide analogues are known and can be used in nucleic acids used in the methods of the invention. A nucleotide analogue is a nucleotide containing a modification at for instance the base, sugar, or phosphate moieties. As an illustrative example, a substitution of 2'-OH residues of siRNA with 2'F, 2'O-Me or 2'H residues is known to improve the *in vivo* stability of the respective RNA. Modifications at the base moiety may be a natural or a synthetic modification of A, C, G, and T/U, a different purine or pyrimidine base, such as uracil-5-yl, hypoxanthin-9-yl, and 2-aminoadenin-9-yl, as well as a non-purine or a non-pyrimidine nucleotide base. Other nucleotide analogues serve as universal bases. Examples of universal bases include 3-nitropyrrole and 5-nitroindole. Universal bases are able to form a base pair with any other base. Base modifications often can be combined with for example a sugar modification, such as for instance 2'-O-methoxyethyl, e.g. to achieve unique properties such as increased duplex stability.

A host cell according to the invention may include an ortholog of the *rhlA* gene, of the *rhlB* gene and/or the *rhlC* gene. An ortholog, or orthologous gene, is a gene with a sequence that has a portion with similarity to a portion of the sequence of a known gene, but found in a different species than the known gene. An ortholog and the known gene originated by vertical descent from a single gene of a common ancestor. As used herein an ortholog encodes a protein that has a portion of at least about 50 %, such as at least about 55 %, at least about 60 %, at least about 65 %, at least about 70 %, at least about 75 %, at least about 80 % or at least about 80 % of the total length of the sequence of the encoded protein that is similar to a portion of a length of at least about 50 %, such as at least about 55 %, at least about 60 %, at least about 65 %, at least about 70 %, at least about 75 %, at least about 80 % or at least about 80 % of a known protein. The respective portion of the ortholog and the respective portion of the known protein to which it is similar may be a continuous sequence or be fragmented into 1 to about 3, including 2, individual regions within the sequence of the respective protein. These 1 to about 3 regions are arranged in the same order in the amino acid sequence of the ortholog and the amino acid sequence of the known protein. Such a portion of an ortholog has an amino acid sequence that has at least about 40 %, at least about 45 %, such as at least about 55 %, at least about 60 %, at least about 65 %, at least about 70 %, at least about 75 % or at least about 80 % sequence identity to the amino acid sequence of the known protein encoded by a *rhlA* gene, a *rhlB* gene or a *rhlC* gene, respectively.

The protein encoded by an ortholog of the *rhlA* gene, the *rhlb* gene or the *rhlC* gene may be identified in a database as a rhamnosyltransferase. An ortholog of a rhamnosyltransferase encoded by an ortholog of a *rhlA* gene may also be be identified as an alpha/beta hydrolase fold protein in a database. An ortholog of a rhamnosyltransferase encoded by an ortholog of a *rhlB* gene may in a database also be identified as a glycosyl transferase. An ortholog of a rhamnosyltransferase encoded by an ortholog of a *rhlC* gene may in a database also be be identified as a rhamnosyltransferase chain C. An ortholog of the *rhlA* gene, the *rhlB* gene or the *rhlC* gene may also be indicated as being of unknown function in a database. Accordingly, a lack of classification as a Rhamnosyltransferase in a database does not exclude a protein with a portion of similar sequence to a known rhamnosyltransferase from being an ortholog.

The heterologous promoters, which may also be addressed as "exogenous" promoters, to which the *rhlA* gene and the *rhlB* gene are operationally linked may be any desired promoter. The term "promoter" as used herein, refers to a nucleic acid sequence needed for gene sequence expression. Promoter regions vary from organism to organism, but are well known to persons skilled in the art for different organisms. For example, in prokaryotes, the promoter region contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence or the CAAT sequence.

The term "heterologous" refers to the relationship between two or more nucleic acid or protein sequences that are derived from different sources. For example, a promoter is heterologous with respect to a transcribable polynucleotide sequence if such a combination is not normally found in nature. In addition, a particular sequence may be "heterologous" with respect to a host cell in that it encodes a protein or is included in a protein, for example a recombinant protein, that is not normally expressed by the host cell. Such a heterologous protein accordingly generally is or has been inserted into the respective host cell, tissue, or species. Accordingly, a heterologous promoter is not normally coupled *in vivo* transcriptionally to the coding sequence of the *rhlA* gene, the *rhlB* gene, or the *rhlC* gene.

In some embodiments the heterologous promoter is a strong promoter. A strong promoter may for example be selected according to the approach disclosed by Dekhtyar et al. (Biotechnol Lett (2010) 32, 243-248) or according to the approach disclosed by Eskin et al. (Pacific Symposium on Biocomputing (2003) 8, 29-40). Illustrative examples of a strong promoter include, but are not limited to, the T7 and the T5 promoters, which are two bacteriophage promoters, the *Escherichia coli* lac promoter, the trc promoter or the tac promoter, which are two functional hybrid promoters derived from the trp and lac promoters, the recA promoter, which is the promoter of a repair protein, the *Escherichia coli* ribosomal RNA rrnB P1 promoter, the adenyl methyltransferase (AMT) promoters AMT-1 and AMT-2, and a synthetic promoter based on the promoter of β-glucanase Pcp7 as disclosed by Spexard et al (Biotechnol Lett (2010) 32, 243-248).

A preferred heterologous promoter is one which confers stronger (higher) expression than the tac promoter, preferably when driving expression of *rhlA*B gene(s). Another particularly preferred promoter is the T7 promoter.

A *rhl*A gene, or an ortholog thereof, may for example be from a *Pseudomonas sp., Burkholderia sp., Enterobacter sp., Pantoea sp., Dickeya sp.,* or *Pantoea sp..* It may for example be from a strain of *Renibacterium salmoninarum, Cellulomonas cellulans, Tetragenococcus* koreensis or *Acinetobacter calcoaceticus.* In some embodiments the *rhlA* gene is from one of *Burkholderia glumae, Burkholderia mallei, Burkholderia pseudomallei, Burkholderia plantarii, Burkholderia gladioli, Burkholderia ubonensis, Burkholderia ambifaria, Burkholderia* cenocepacia, *Burkholderia caryophylli, Dickeya zeae, Pseudomonas aeruginosa, Pseudomonas fluorescens, Pseudomonas putida, Pseudomonas oleovorans, Pseudomonas chlororaphis, Pantoea stewartii, Pseudomonas mendocina, Pseudomonas nitroreducens, Pseudomonas entomophila, Pseudomonas brassicacearum, Pseudomonas stutzeri, Pseudomonas* fluorescens, *Pseudomonas oleovorans, Pantoea ananatis, Serratia odorifera, Halothiobacillus neapolitanus, Enterobacter asburiae* and *Enterobacter hormaechei.*

A *rhlB* gene, or an ortholog thereof, may be from a bacterium of one of the clases Alphaproteobacteria, Betaproteobacteria, Gammaproteobacteria, Planctomycetacia, Acidobacteriales and Solibacteres. A *rhlB* gene, or an ortholog thereof, may for example be from a *Pseudomonas sp., Burkholderia sp., Enterobacter sp., Pantoea sp., Dickeya sp., Blastopirellula sp., Pantoea sp., Methylobacterium sp.,* or *Acidobacterium sp..* In some embodiments the *rhlB* gene is from one of *Pseudomonas aeruginosa, Burkholderia glumae, Burkholderia mallei, Burkholderia pseudomallei, Burkholderia thailandensis, Burkholderia ambifaria, Burkholderia cepacia, Burkholderia cenocepacia, Burkholderia gladioli, Dickeya dadantii, Pantoea ananatis, Planctomyces limnophilus, Blastopirellula marina, Methylobacterium extorquens, Methylobacterium chloromethanicum, Maritimibacter alkaliphilus, Acidobacterium capsulatum* and *Solibacter usitatus.*

A *rhlC* gene, or an ortholog thereof, may be from a bacterium of one of the classes Alphaproteobacteria, Betaproteobacteria, Gammaproteobacteria, Acidobacteriales, and Planctomycetacia. A *rhlC* gene, or an ortholog thereof, may for example be from one of *Pseudomonas aeruginosa, Ralstonia solanacearum, Burkholderia glumae, Burkholderia pseudomallei, Burkholderia mallei, Burkholderia thailandensis, Burkholderia gladioli, Burkholderia cenocepacia, Burkholderia ambifaria, Burkholderia phytofirmans, Burkholderia phymatum, Burkholderia multivorans, Lautropia mirabilis, Variovorax paradoxus, Methylobacterium populi, Methylobacterium chloromethanicum, Methylobacterium extorquens, Methylotenera mobilis* and *Planctomyces maris.* The source of the *rhlA* gene, or the ortholog thereof, of the *rhlB* gene, or the ortholog thereof and, where present, of the *rhlC* gene, or the ortholog thereof are independently selected. In some embodiments the *rhlA* gene, or the ortholog thereof, and the *rhlB* gene, or the ortholog thereof, are from the same organism, e.g. the same species or the same strain. In some embodiments the *rhlA* gene, or the ortholog thereof, and the *rhlB* gene, or the ortholog thereof, are from different organisms, e.g. different species or different strains. The selection of the *rhlA* gene, or the ortholog thereof, and the *rhlB* gene, or the ortholog thereof, may affect the structure of the rhamnolipds produced by the bacterial host cell. Andrä et al. (Biol. Chem. (2006) 387, 301-310) and Hörmann et al. (Eur. J. Lipid Sci. Technol. (2010) 112, 674-680) have for example reported that B. plantarii, strains DSM 6535 and DSM 9509 produce a dirhamnolipid with two saturated 3-hydroxy-n-tetradecanoic acid fatty acid chains, whereas P. aeruginosa produces a dirhamnolipid with two saturated 3-hydroxy-n-decanoic acid fatty acid chains.

In some embodiments of a method of the invention a host cell is allowed to express a biosurfactant such as a glycolipid, which may for instance be a rhamnolipid (supra). As explained above, the host cell may include a nucleic acid molecule encoding a respective biosurfactant and an operably linked promoter. Such a nucleic acid molecule may have been introduced or be introduced into a recipient cell such as a prokaryotic cell either as a nonreplicating DNA or RNA molecule, which may be a linear molecule or a closed covalent circular molecule. Since such molecules are incapable of autonomous replication, the expression of the gene may occur through the transient expression of the introduced sequence. In some embodiments, permanent expression may occur through the integration of the introduced DNA sequence into a host chromosome.

A vector may be employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotrophic host, biocide resistance, e.g., antibiotics, or heavy metals, such as copper, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals.

The introduced nucleic acid molecule can be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

An illustrative example of a prokaryotic vector is a plasmid, such as a plasmid capable of replication in E. *coli* (such as, for example, pBR322, CoIEI, pSC101, pACYC 184, VX). Bacillus plasmids include pC194, pC221, pT127, and the like. Suitable Streptomyces plasmids include p1J101 (Kendall et al., J. Bacteriol. (1987) 169, 4177-4183), and streptomyces bacteriophages such as C31. *Pseudomonas* plasmids are for instance reviewed by John et al. (Rev. Infect. Dis. 8:693-704, 1986).

Once the vector or nucleic acid molecule that contains the construct(s) has been prepared for expression, the DNA construct(s) may be introduced into the host cell by any of a variety of suitable means, i.e., transformation, transfection, conjugation, protoplast fusion, electroporation, particle gun technology, calcium phosphate-precipitation, direct microinjection, and the like. After the introduction of the vector, recipient cells are grown in a selective medium, which selects for the growth of vector-containing cells. Expression of the cloned gene(s) results in the production of a kinase of the invention, or fragments thereof. This can take place in the transformed cells as such, or following the induction of these cells to differentiate. A variety of incubation conditions can be used to form the peptide of the present invention. It may be desired to use conditions thatmimic physiological conditions.

The terms "expression" and "expressed", as used herein, are used in their broadest meaning, to signify that a sequence included in a nucleic acid molecule and encoding a peptide/protein is converted into its peptide/protein product. Thus, where the nucleic acid is DNA, expression refers to the transcription of a sequence of the DNA into RNA and the translation of the RNA into protein. Where the nucleic acid is RNA, expression may include the replication of this RNA into further RNA copies and/or the reverse transcription of the RNA into DNA and optionally the transcription of this DNA into further RNA molecule(s). In any case expression of RNA includes the translation of any of the RNA species provided/produced into protein. Hence, expression is performed by translation and includes one or more processes selected from the group consisting of transcription, reverse transcription and replication. Expression of the protein or peptide of the member of the plurality of peptides and/ or proteins may be carried out using an in vitro expression system. Such an expression system may include a cell extract, typically from bacteria, rabbit reticulocytes or wheat germ. Many suitable systems are commercially available. The mixture of amino acids used may include synthetic amino acids if desired, to increase the possible number or variety of proteins produced in the library. This can be accomplished by charging tRNAs with artificial amino acids and using these tRNAs for the in vitro translation of the proteins to be selected. A nucleic acid molecule, such as DNA, is said to be "capable of expressing" a peptide/protein if it contains nucleotide sequences which contain transcriptional and translational regulatory information and such sequences are operably linked to nucleotide sequences which encode the polypeptide. A suitable embodiment for expression purposes is the use of a vector, in particular an expression vector. Thus, the present invention also provides a host cell transformed/transfected with an expression vector.

An expression vector, which may include one or more regulatory sequences and be capable of directing the expression of nucleic acids to which it is operably linked. An operable linkage is a linkage in which a coding nucleotide sequence of interest is linked to one or more regulatory sequence(s) such that expression of the nucleotide sequence sought to be expressed can be allowed. Thus, a regulatory sequence operably linked to a coding sequence is capable of effecting the expression of the coding sequence, for instance in an in vitro transcription/ translation system or in a cell when the vector is introduced into the cell. A respective regulatory sequence need not be contiguous with the coding sequence, as long as it functions to direct the expression thereof. Thus, for example, intervening untranslated yet transcribed sequences may be present between a promoter sequence and the coding sequence and the promoter sequence can still be considered "operably linked" to the coding sequence.

The term "regulatory sequence" includes controllable transcriptional promoters, operators, enhancers, silencers, transcriptional terminators, 5' and 3' untranslated regions which interact with host cellular proteins to carry out transcription and translation and other elements that may control gene expression including initiation and termination codons. The regulatory sequences can be native (homologous), or can be foreign (heterologous) to the cell and/or the nucleotide sequence that is used. The precise nature of the regulatory sequences needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence or CAAT sequence. These regulatory sequences are generally individually selected for a certain embodiment, for example for a certain cell to be used. The skilled artisan will be aware that proper expression in a prokaryotic cell also requires the presence of a ribosome-binding site upstream of the gene sequence-encoding sequence.

The term "transfecting" defines a number of methods to insert a nucleic acid vector or other nucleic acid molecules into a cellular organism. These methods involve a variety of techniques, such as treating the cells with high concentrations of salt, an electric field, detergent, or DMSO to render the outer membrane or wall of the cells permeable to nucleic acid molecules of interest or use of various viral transduction strategies.

In an embodiment of a method according to the invention a host cell is cultured under conditions that allow rhamnolipid production. Suitable conditions are within the routine knowledge of the skilled person. The formation of rhamnolipids can further be easily analysed and/or monitored since rhamnolipids are generally being secreted by a host cell. Accordingly, standard techniques of cell culture broth analysis, including chromatographic techniques such as HPLC, can be applied in this regard. Suitable conditions for culturing the host cell typically include culturing the same in an aqueous medium that is suitable for sustaining cell viability and cell growth. Illustrative examples of a suitable cell culture medium, for example for culturing a bacterial host such as a Pseudomonas sp. host or a Burkholderia sp. host, include, but are not limited to, Luria-Bertani (LB) complex medium, Inkas-medium, phosphate-limited protease peptone-glucose-ammonium salt medium (PPGAS), Minimal medium E (MME). In some embodiments, the media used may include a factor selected from growth factors and/or attachment factors. In some embodiments the media used may be void of such a factor. In some embodiments it may be sufficient to add such a factor only to the media used for the seeding of the cells and/or the growing of the cells, for example under logarithmic conditions. In some embodiments serum may be included in a media used. In some embodiments the media may be serum-free, i.e. void of any sera from animal or human origin. Suitable cell culture media may further include salts, vitamins, buffers, energy sources, amino acids and other substances.

The temperature applied in a method of producing a biosurfactant, preferably a rhamnolipid is at or above 30°C. In particular, the temperature when producing a rhamnolipid is above 30°C.

In other embodiments, the temperature applied in the methods of the present invention may be about or at temperature of 31 °C, 32°C, 33°C, 34°C, 35°C, 36°C or 37°C, with about or at a temperature of 32°C, 33°C or 34°C being preferred. A temperature about or at 33°C is even more preferred.

Alternatively, the temperature may be in a range of more than (>) 30°C - 37°C, 30°C - 36°C, 30°C - 35°C, 30°C - 34°C, 30°C - 33°C, 30°C - 32°C, 30°C - 31°C, with a range of more than (>) 30°C - 35°C, 30°C - 34°C, 30°C - 33°C being preferred.

In a yet further alternative, the temperature may be in a range of 31°C - 37°C, 31°C - 36°C, 31°C - 35°C, 31°C - 34°C, 31°C - 33°C, 31°C - 32°C, with a range of 31°C - 35°C, 31°C - 34°C, 31°C - 33°C being preferred.

In a further aspect the present disclosure concerns a biosurfactant obtainable by a method of the present invention. The biosurfactant is preferably one described herein, in particular a rhamnolipid.

### FIGURES

The Figures show:
Figure 1 : Concept of the adsorption column connected to the bioreactor
Figure 2: Design of the adsorber column

### EXAMPLES

The invention will now be described by reference to the following Examples which are merely illustrative and are not construed as a limitation of the scope the present invention.

### Example 1

A genetically modified *Pseudomonas putida* strain is cultivated in a 3.2 liter fermentor KLF2000 (Bioengineering AG, Wald, Zh, CH) with a working volume of about 2 liters. Precultures from 100 - 200 ml scale from shaker flasks were used for inoculation of the fermentor. The medium (LB-Medium supplemented with Glucose) is autoclaved in-situ and aerated by sterile-filtered air. The stirring is maintained by two 6-blade Rushton-impeller stirrers. pH control is done by addition of 2 M HCl or 2 M NaOH. The pO₂ is controlled primarily by regulation of the stirrer speed (up to 800 rpm) and aeration (up to 100 l/h) and is held above 10%. For feeding of glucose during the fermentation a peristaltic pump is connected to a flask containing a glucose solution of 400 g/l. After inoculation of the fermentor with the preculture and the subsequent growth phase (3-5 hours) foam is formed. The supplied air is finely dispersed by the stirrer in the fermentor to allow a sufficient supply of oxygen for the growing microorganisms. During the growth of the microorganisms rhamnolipids are formed which are amphiphilic compounds. The surface of the air bubbles attracts the amphiphilic rhamnolipid molecules which then adhere to their surface. After the gas bubbles have risen to the surface of the fermentation medium the adhered rhamnolipid molecules stabilize the gas bubbles so that they form foam. With more and more foam bubbles formed, this foam is foaming out of the top gas outlet of the fermentor. This foam is directly led into a column filled with a hydrophobic adsorber resin (see Figure 1).

In a steel column hydrophobic adsorber resins like XAD-2 or XAD1600N (Rohm&Haas, USA) then strongly bind the hydrophobic moiety of the rhamnolipids on their surface. Due to this the foam is destroyed and at the end of the bed of the adsorber resin fermentation liquid contained in the lamella of the foam bubbles can be separated from the air. The column used is of a proprietary design and consists of a distribution unit for the foam, a height-adjustable device for the resin as well as an outlet for the gas / liquid mixture (Figure 2). Besides the rhamnolipid the liquid lamella of the foam contains fermentation medium from which cells partly also are adsorbed on the resin.

After the loading capacity of the resin is reached, the column is switched from the fermentor exhaust line and first any adsorbed cells are washed off from the resin with water. The desorption of the adsorbed rhamnolipid is done using methanol. The methanol solution is sterile filtered and freeze-dried to yield a brownish powder mainly consisting out of rhamnolipid.

| Component used | Company | Concentration |
|---|---|---|
| LB-Media (Lennox) | Roth | 20 g/l |
| Tetracyclin hydrochloride | AppliChem | 50 µM |
| Glucose monohydrate | Sigma-Aldrich | 10 g/l |
| Hydrochloric acid | Merck | 2 M |
| Sodium hydroxide | Roth | 2 M |

## Claims

1. A method for the isolation of an amphipathic compound from a hydrophobic or hydrophilic medium comprising
- allowing the formation and/or accumulation of foam comprising said amphipathic compound at the hydrophobic or hydrophilic medium-gas interface, respectively,
- applying said foam directly onto an adsorbent which effects collapse of said foam, and
- isolating said adsorbed amphipathic compound by desorption,
wherein said adsorbent is hydrophobic if the solvent of the culture broth is hydrophilic,
wherein said adsorbent is hydrophilic if the solvent of the culture broth is hydrophobic,
wherein the adsorbent is in the form of particles,
optionally comprising one or more further steps of purifying said amphipathic compound.

2. A method for the isolation of a hydrophobic compound from a hydrophilic medium comprising
- allowing the formation and/or accumulation of foam comprising said hydrophobic compound at the hydrophilic medium-gas interface,
- applying said foam directly onto an adsorbent which effects collapse of said foam, and
- isolating said adsorbed hydrophobic compound by desorption,
wherein said adsorbent is hydrophobic,
wherein the adsorbent is in the form of particles,
optionally comprising one or more further steps of purifying said hydrophobic compound.

3. A method for the isolation of a hydrophilic compound from a hydrophobic medium comprising
- allowing the formation and/or accumulation of foam comprising said hydrophilic compound at the hydrophobic medium-gas interface,
- applying said foam directly onto an adsorbent which effects collapse of said foam, and
- isolating said adsorbed hydrophilic compound by desorption,
wherein said adsorbent is hydrophilic,
wherein the adsorbent is in the form of particles,
optionally comprising one or more further steps of purifying said hydrophilic compound.

4. The method of claim 1, wherein said amphipathic compound is a biosurfactant.

5. The method of claim 1 or 4, wherein said amphipathic compound is produced by culturing a host cell capable of producing said biosurfactant.

6. The method of any one of claims 1, 4 or 5 which is for the *in situ* isolation of a biosurfactant comprising
(a) culturing a host cell capable of producing said biosurfactant,
(b) allowing the formation and/or accumulation of foam comprising said biosurfactant at the culture broth-gas interface,
(c) applying said foam directly onto an adsorbent which effects collapse of said foam, and
(d) isolating said adsorbed biosurfactant by desorption,
optionally comprising one or more further steps of purifying said biosurfactant.

7. The method of any one the preceding claims, wherein said adsorbent is adsorbent material in bulk.

8. The method of claim 6 or 7, wherein said cell is a prokaryotic cell.

9. The method of claim 8, wherein said cell comprises
(i) a *rhlA* gene or an ortholog thereof; and
(ii) a *rhlB* gene or an ortholog thereof.

10. The method of claim 9, wherein the cell further comprises a *rhlC* gene or an ortholog thereof.

11. The method of any one of claims 6 or 8 to 10, wherein said cell is grown at a temperature above 30°C.

## Patentansprüche

1. Verfahren zur Isolation einer amphipathischen Verbindung aus einem hydrophobem oder hydrophilen Medium umfassend
- Bildenlassen und/oder Akkumulierenlassen von Schaum umfassend die amphipathische Verbindung an der hydrophoben oder hydrophilen Medium-Gas-Grenzfläche, entsprechend,
- Auftragen des Schaums direkt auf ein Adsorbens, das das Kollabieren des Schaums bewirkt, und
- Isolieren der adsorbierten amphipathischen Verbindung durch Desorption,
wobei das Adsorbens hydrophob ist, wenn das Lösungsmittel der Kulturbrühe hydrophil ist,
wobei das Adsorbens hydrophil ist, wenn das Lösungsmittel der Kulturbrühe hydrophob ist,
wobei das Adsorbens in Partikelform ist,
optional umfassend einen oder mehrere weitere(n) Schritt(e) der Aufreinigung der amphipathischen Verbindung.

2. Verfahren zur Isolation einer hydrophoben Verbindung aus einem hydrophilen Medium umfassend
- Bildenlassen und/oder Akkumulierenlassen von Schaum umfassend die hydrophobe Verbindung an der hydrophilen Medium-Gas-Grenzfläche,
- Auftragen des Schaums direkt auf ein Adsorbens, das das Kollabieren des Schaums bewirkt, und
- Isolieren der adsorbierten hydrophoben Verbindung durch Desorption,
wobei das Adsorbens hydrophob ist,
wobei das Adsorbens in Partikelform ist,
optional umfassend einen oder mehrere weitere(n) Schritt(e) der Aufreinigung der hydrophoben Verbindung.

3. Verfahren zur Isolation einer hydrophilen Verbindung aus einem hydrophoben Medium umfassend
- Bildenlassen und/oder Akkumulierenlassen von Schaum umfassend die hydrophile Verbindung an der hydrophoben Medium-Gas-Grenzfläche,
- Auftragen des Schaums direkt auf ein Adsorbens, das das Kollabieren des Schaums bewirkt, und
- Isolieren der adsorbierten hydrophilen Verbindung durch Desorption,
wobei das Adsorbens hydrophil ist,
wobei das Adsorbens in Partikelform ist,
optional umfassend einen oder mehrere weitere(n) Schritt(e) der Aufreinigung der hydrophoben Verbindung.

4. Verfahren nach Anspruch 1, wobei die amphipathische Verbindung ein Biotensid ist.

5. Verfahren nach Anspruch 1 oder 4, wobei die amphipathische Verbindung hergestellt wird durch Kultivieren einer Wirtszelle, die in der Lage ist, das Biotensid herzustellen.

6. Verfahren nach einem der Ansprüche 1, 4 oder 5, das für die *in situ*-Isolation eines Biotensids ist, umfassend
(a) Kultivieren einer Wirtszelle, die in der Lage ist, das Biotensid herzustellen;
(b) Bildenlassen und/oder Akkumulierenlassen von Schaum umfassend das Biotensid an der Kulturbrühe-Gas-Grenzfläche;
(c) Auftragen des Schaums direkt auf das Adsorbens, das das Kollabieren des Schaums bewirkt; und
(d) Isolieren des adsorbierten Biotensids durch Desorption,
optional umfassend einen oder mehrere weitere(n) Schritt(e) der Aufreinigung des Biotensids.

7. Verfahren nach einem der vorhergehenden Ansprüche, wobei das Adsorbens ein loses Adsorbensmaterial ist.

8. Verfahren nach Anspruch 6 oder 7, wobei die Zelle eine prokaryontische Zelle ist.

9. Verfahren nach Anspruch 8, wobei die Zelle umfasst
(i) ein *rhlA-*Gen oder ein Ortholog davon; und
(ii) ein *rhlB*-Gen oder ein Ortholog davon.

10. Verfahren nach Anspruch 9, wobei die Zelle weiterhin ein *rhlC*-Gen oder ein Ortholog davon umfasst.

11. Verfahren nach einem Ansprüche 6 oder 8 bis 10, wobei die Zelle bei einer Temperatur oberhalb von 30 °C gezüchtet wird.

## Revendications

1. Procédé d'isolation d'un composé amphipathique provenant d'un milieu hydrophobe ou hydrophile consistant à
permettre la formation et/ou l'accumulation de mousse comprenant ledit composé amphipathique au niveau de l'interface gaz/milieu hydrophobe ou hydrophile, respectivement,
appliquer ladite mousse directement sur un adsorbant qui a pour effet d'affaisser ladite mousse, et
isoler ledit composé amphipathique adsorbé par désorption,
ledit adsorbant étant hydrophobe si le solvant du bouillon de culture est hydrophile,
ledit adsorbant étant hydrophile si le solvant du bouillon de culture est hydrophobe,
l'adsorbant se présentant sous forme de particules,
comprenant éventuellement une ou plusieurs autres étapes de purification dudit composé amphipathique.

2. Procédé d'isolation d'un composé hydrophobique provenant d'un support hydrophile consistant à
permettre la formation et/ou l'accumulation de mousse comprenant ledit composé hydrophobique au niveau de l'interface gaz/support hydrophile, appliquer ladite mousse directement sur un adsorbant qui a pour effet d'affaisser ladite mousse, et
isoler ledit composé hydrophobe adsorbé par désorption,
ledit adsorbant étant hydrophobe,
l'adsorbant se présentant sous forme de particules,
comprenant éventuellement une ou plusieurs autres étapes de purification dudit composé hydrophobe.

3. Procédé d'isolation d'un composé hydrophile provenant d'un milieu hydrophobe consistant à
permettre la formation et/ou l'accumulation de mousse comprenant ledit composé hydrophile au niveau de l'interface gaz/milieu hydrophobe,
appliquer ladite mousse directement sur un adsorbant qui a pour effet d'affaisser ladite mousse, et
isoler ledit composé adsorbé hydrophile par désorption, ledit adsorbant étant hydrophile,
l'adsorbant se présentant sous forme de particules,
comprenant éventuellement une ou plusieurs autres étapes de purification dudit composé hydrophile.

4. Procédé selon la revendication 1, selon lequel ledit composé amphipathique est un biosurfactif.

5. Procédé selon la revendication 1 ou la revendication 4, selon lequel ledit composé amphipathique est produit par la culture d'une cellule hôte capable de produire ledit biosurfactif.

6. Procédé selon l'une quelconque des revendications 1, 4 ou 5 qui sert pour l'isolation in situ d'un biosurfactif consistant à
(a) cultiver une cellule hôte capable de produire ledit biosurfactif
(b) permettre la formation et/ou l'accumulation de mousse comprenant ledit biosurfactif au niveau de l'interface gaz/bouillon de culture,
(c) appliquer ladite mousse directement sur un adsorbant qui a pour effet d'affaisser ladite mousse, et
(d) isoler ledit biosurfactif adsorbé par désorption,
comprenant éventuellement une ou plusieurs autres étapes de purification dudit biosurfactif.

7. Procédé selon l'une quelconque des revendications précédentes, selon lequel ledit adsorbant est un matériau adsorbant en vrac.

8. Procédé selon la revendication 6 ou 7, selon lequel ladite cellule est une cellule procaryote.

9. Procédé selon la revendication 8, selon lequel ladite cellule comprend
(i) un gène *rh*/*A* ou son orthologue ; et
(i) un gène *rh*/*B* ou son orthologue.

10. Procédé selon la revendication 9, selon lequel la cellule comprend en outre un gène *rh*/*C* ou son orthologue.

11. Procédé selon l'une quelconque des revendications 6 ou 8 à 10, selon lequel ladite cellule est cultivée à une température supérieure à 30°C.
